(19) Europäisches Patentamt

European Patent Office

Office européen des brevets

(11) EP 1 062 975 B1

(12) EUROPÄISCHE PATENTSCHRIFT

(45) Veröffentlichungstag und Bekanntmachung des Hinweises auf die Patenterteilung:
**21.06.2006 Patentblatt 2006/25**

(51) Int Cl.:
***A61N 1/365*** (2006.01)

(21) Anmeldenummer: **00250208.6**

(22) Anmeldetag: **23.06.2000**

(54) **Kardioelektrische Vorrichtung zum vorzeitigen Erkennen einer Tachycardie**

Cardio-electrical device for early detection of tachycardia

Appareil cardio-électrique de reconnaissance précoce de tachycardie

(84) Benannte Vertragsstaaten:
**AT BE CH CY DE DK ES FI FR GB GR IE IT LI LU MC NL PT SE**

(30) Priorität: **25.06.1999 DE 19930270**

(43) Veröffentlichungstag der Anmeldung:
**27.12.2000 Patentblatt 2000/52**

(73) Patentinhaber: **BIOTRONIK GmbH & Co. KG**
**12359 Berlin (DE)**

(72) Erfinder: **Weiss, Ingo**
**91056 Erlangen (DE)**

(74) Vertreter: **Lindner-Vogt, Karin L. et al**
**Biotronik GmbH & Co. KG**
**Woermannkehre 1**
**12359 Berlin (DE)**

(56) Entgegenhaltungen:
**US-A- 4 830 006**

- **DATABASE BIOSIS [Online] BIOSCIENCES INFORMATION SERVICE, PHILADELPHIA, PA, US; KIRCHHOF PAULUS ET AL: "Postrepolarization refractoriness versus conduction slowing caused by class I antiarrhytmic drugs: antiarrhytmic and proarrhythmic effects" XP002262781**

**Beschreibung**

Die Erfindung betrifft eine Vorrichtung einer Tachykardie eines Herzens.

Ein Herz, insbesondere ein menschliches Herz, kann in physiologisch bedenkliche und möglicherweise tödliche Zustände geraten. Ein solcher Zustand ist eine sogenannte Tachykardie, die sich durch sehr schnell aufeinanderfolgende Herzschläge, also eine hohe Herzrate bei gleichzeitig verminderter Pumpleistung des Herzens auszeichnet.

Vorrichtungen zum Erkennen und Behandeln solcher Tachykardien, beispielsweise Kardioverter oder Defibrillatoren, sind grundsätzlich bekannt. Ein Nachteil der bekannten Vorrichtung besteht darin, daß sie eine Tachykardie erst dann erkennen, wenn, wenn sie bereits eingetreten ist.

Ziel der vorliegenden Erfindung ist es, eine Vorrichtung anzugeben, die in der Lage ist, eine Tachykardie vorzeitig, d.h. vor Beginn der Tachykardie zu erkennen. Erfindungsgemäß wird diese Aufgabe mit einer Vorrichtung zum vorzeitigen Erkennen einer Tachykardie eines Herzens gelöst, die folgende Bestandteile umfaßt:

- Meßmittel zum Aufnehmen von Meßwerten für die Herzrate und die Aktionspotentialdauer, mit mindestens einem Ausgang zum Ausgeben vom Meßwertpaaren einander zugeordneter Meßwerte für die Herzrate und Aktionspotentialdauer,

- Meßwertverarbeitungsmittel, die zur Übernahme der Meßwertpaare mit dem Ausgang der Meßmittel verbunden sind und zum Ableiten das Herz beschreibender, zeitvarianter Parameter aus den Meßwertpaaren ausgebildet sind,

- einen Speicher, in dem eine Tachykardiegefahr kennzeichnende Vergleichswerte speicherbar sind und

- eine Auswerteeinheit, die mit den Meßwertverarbeitungsmitteln zur bernahme der aus den Meßwertpaaren abgeleiteten Parameter und mit dem Speicher verbunden ist, und die zum Vergleichen der abgeleiteten Parameter mit im Speicher gespeicherten Vergleichswerten und zum Ausgeben eines Tachykardiegefahrensignals ausgebildet ist, falls der Vergleich der abgeleiteten Parameter mit den Vergleichswerten ergibt, daß die abgeleiteten Parameter in dem Tachykardiegefahrenbereich liegen.

Eine derartige Vorrichtung ist in der Tat in der Lage, eine drohende Tachykardie zu erkennen, bevor sie eintritt. WO-A-98 32 489 beschreibt eine derartige Vorrichtung zur Froherkennung von Tachykardie. Die Ableitung das Herz beschreibender, zeitvarianter Parameter aus den Meßwertpaaren wird im folgenden ausführlich beschrieben werden. Das Bestimmen der Herzrate, also der Pulsfrequenz des Herzens, sowie der Aktionspotentialdauer ist für sich genommen allgemein bekannt, wie sich aus der folgenden Beschreibung ebenfalls ergibt. Außerdem ist es grundsätzlich bekannt, wie eine Vorrichtung gestaltet sein muß, daß sie in der Lage ist, festzustellen, ob bestimmte Parameter in einen bestimmten Parameterbereich fallen.

Bevorzugt wird eine Vorrichtung, die zum Aufnehmen mindestens zweier Meßwertepaare zu verschiedenen Meßzeitpunkten ausgebildet ist und zusätzlich einen zweiten Speicher, der mit den Meßwertverarbeitungsmitteln verbunden ist und in dem zumindest die für die zwei jeweils jüngsten Meßzeitpunkte abgeleiteten Parameter speicherbar sind, umfaßt, sowie Trendbestimmungsmittel, die mit dem zweiten Speicher verbunden sind und die zum Bestimmen eines Trendes für die zukünftige Entwicklung der Parameter anhand der gespeicherten Parameter ausgebildet sind, wobei die Auswerteeinheit mit den Trendbestimmungsmitteln verbunden und so ausgebildet ist, daß sie das Tachykardiegefahrensignal ausgibt, wenn die abgeleiteten Parameter in der Nähe des Tachykardiegefahrenbereichs liegen und der Trend für die zukünftige Entwicklung der Parameter in Richtung des Tachykardiegefahrenbereichs weist. Eine derartige Vorrichtung ist in der Lage, eine Tachykardiegefahr zu erkennen, bevor die ermittelten Parameter in einen als gefährlich bekannten Bereich fallen, da die Vorrichtung bereits vorher ermitteln kann, daß sich die aus den Meßwertpaaren abgeleiteten Parameter in Richtung des Gefahrenbereichs entwickeln.

Zu diesem Zweck dient auch eine alternativ bevorzugte Vorrichtung, die zum Aufnehmen mindestens zweier Meßwertpaare zu verschiedenen Meßzeitpunkten ausgebildet ist und zusätzlich einen zweiten Speicher, der mit den Meßwertverarbeitungsmitteln verbunden ist und in dem zumindest für die zwei jeweils jüngsten Meßzeitpunkte abgeleiteten Parameter speicherbar sind, umfaßt, sowie Trendbestimmungsmittel, die mit dem zweiten Speicher verbunden sind und die zum Extrapolieren zukünftiger Parameter aus den gespeicherten Parametern ausgebildet sind, wobei die Auswerteeinheit mit den Trendbestimmungsmitteln verbunden und zum Vergleichen der extrapolierten Parameter mit im Speicher gespeicherten Vergleichswerten und zum Ausgeben eines Tachykardiegefahrensignals ausgebildet sind, falls der Vergleich der extrapolierten Parameter mit den Vergleichswerten ergibt, daß die extrapolierten Parameter in dem Tachykardiegefahrenbereich liegen.

Besonders bevorzugt ist eine Vorrichtung, bei der die abgeleiteten Parameter zeitvariable, bidirektionale Ionenströme zwischen dem Zellinneren und dem Zelläußeren von Herzmuskelzellen beschreiben, zu denen einen statischer und ein dynamischer Kalium-Ionenstrom sowie einen Kalziumionenstrom zählen, wobei ein erster der Parameter

($\tau_x$) eine Zeitkonstante des dynamischen Kalium-Ionenstroms ist und ein zweiter der Parameter (g) eine auf die Amplitude des dynamischen Kalium-Ionenstroms ($A_{Kx}$) bezogene Differenz ($A_{Ca} - A_{K1}$) der Amplituden des Kalzium-Ionenstroms ($A_{Ca}$) und des statischen Kalium-Ionenstroms ($A_{Kl}$) ist:

$$g = ((A_{Ca} - A_{Kl}) / A_{Kx}).$$

Wie sich aus der folgenden ausführlichen Beschreibung ergibt, sind diese Parameter geeignet, die Eigenschaften von Herzmuskelzellen so zu beschreiben, daß eine vorzeitige Tachykardieerkennung möglich ist.

In einer alternativen Begrifflichkeit läßt sich der vorbeschriebene Sachverhalt auch so ausdrücken, daß der erste Parameter ($\tau_x$) die Zeitkonstante des dynamischen Kaliumkanals der Herzmuskelzellen und der zweite Parameter (g) die Verhältnisse (($A_{Ca} - A_{Kl}$) / ($A_{Kx}$)) zwischen den Permeabilitäten des Kalziumkanals ($A_{Ca}$) und des statischen Kaliumkanals ($A_{Kl}$) sowie des dynamischen Kaliumkanals ($A_{Kx}$) der Herzmuskelzellen repräsentieren.

Besonders bevorzugt ist eine Vorrichtung, die zusätzlich einen Elektrostimulator wie einen Kardioverter oder einen Defibrillator aufweist, und die sich dadurch auszeichnet, daß der Elektrostimulator mit Auswerteeinheit zur Übernahme des Tachykardiegefahrensignals verbunden und durch das Tachykardiegefahrensignal zur Abgabe tachykardiebekämpfender elektrischer Stimulationsimpulse an das Herz auslösbar gestaltet ist.

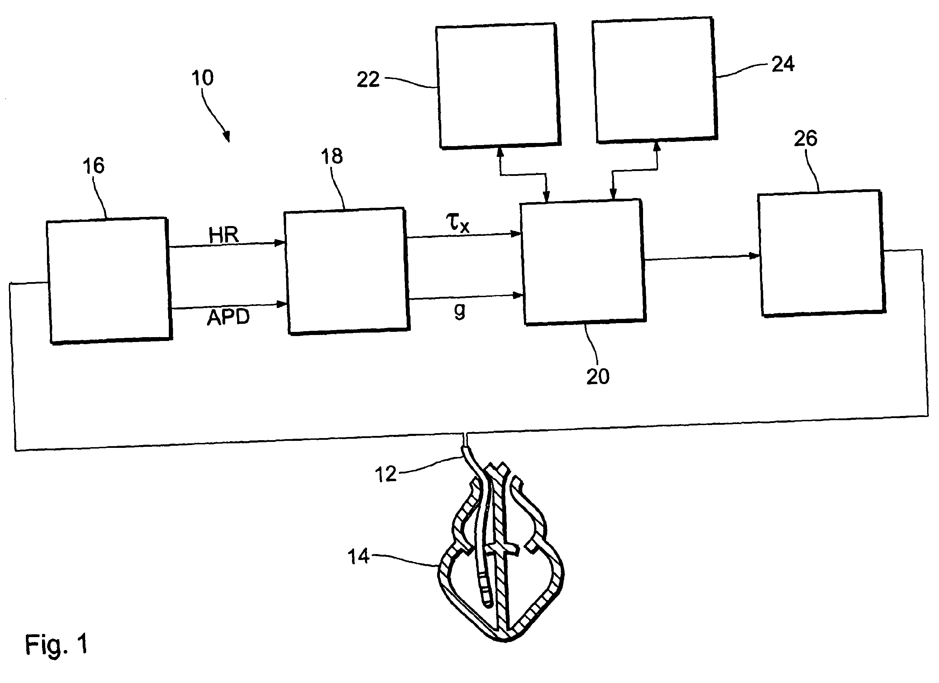

Die Erfindung soll nun anhand eines Ausführungsbeispiels mit Hilfe der Figur näher erläutert werden. Fig. 1 zeigt eine Vorrichtung zum vorzeitigen Erkennen einer Tachykardie eines Herzens.

Diese Vorrichtung 10 umfaßt eine Elektrodenleitung 12, die zur Aufnahme elektrischer Signale eines Herzens 14 mit einer Meßeinheit 16 verbunden ist. Die Meßmittel 16 leiten aus den aufgenommenen elektrischen Signalen Meßwerte für die Herzrate HR des Herzens 14 bzw. den Kehrwert der Herzrate HR, die Zykluslänge L einerseits und andererseits Meßwerte für die Depolarisationszeit T bzw. die Aktionspotentialdauer APD des Herzens ab. Diese Meßwerte werden zu verschiedenen Zeitpunkten ermittelt und paarweise einander zugeordnet an eine Meßwertverarbeitungseinheit 18 weitergeleitet. Die Meßwertverarbeitungseinheit 18 ist dazu eingangsseitig mit entsprechenden Ausgängen der Meßeinheit 16 verbunden.

In der Meßwertverarbeitungseinheit 18 werden aus der Depolarisationszeit T bzw. der Aktionspotentialdauer APD und der Herzrate HR bzw. deren Kehrwert, der Zykluslänge L eine Zeitkonstante $\tau_x$ des dynamischen Kalium-Kanals der Herzmuskelzellen des Herzens 14 sowie eine Differenz g der Kalzium-Stromamplitude $A_{Ca}$ und der Kaliumstromamplitude $A_{Kl}$ bezogen auf die Amplitude des dynamischen Kaliumstroms $A_{Kx}$ gebildet. Die Differenz g stellt dabei die Verhältnisse der Permeabilitäten des Kalzium- und der Kaliumkanäle dar. Der Ableitung der Parameter g und $\tau_x$ liegt das im folgenden beschriebene Modell eines Herzens als zeitvariables System zugrunde, in dem $\tau_x$ und g zeitvariante Strukturparameter sind. Die Ableitung dieser Parameter erfolgt durch die beschriebene Linearisierung, wobei von einem gewählten Linearisierungspunkt innerhalb des Modells ausgegangen wird und eine iterative Annäherung an den tatsächlichen Arbeitspunkt des Herzens erfolgt. Die Einzelheiten sind weiter hinten näher beschrieben.

Die abgeleiteten Parameter $\tau_x$ und g werden von der Meßwertverarbeitungseinheit 18 als einander zugeordnete Wertepaare an eine Auswerteeinheit 20 weitergeleitet, die dazu eingangsseitig mit dem Ausgang der Meßwertverarbeitungseinheit 18 verbunden ist. Außerdem ist die Auswerteeinheit 20 mit einem ersten Speicher 22 und einem zweiten Speicher 24 verbunden, die eine physikalische Einheit bilden können. In dem ersten Speicher 22 sind Vergleichswerte für die Parameter $\tau_x$ und g gespeichert, die eine Gefahrenzone oder einen Tachykardiegefahrenbereich kennzeichnen, d.h. Vergleichswerte für solche Wertepaare der Parameter $\tau_x$ und g, die eine Tachykardiegefahr indizieren. Die Auswerteeinheit 20 ist dabei so ausgebildet, daß sie die abgeleiteten und von der Meßwertverarbeitungseinheit 18 empfangenen Werte für die Parameter $\tau_x$ und g mit den in dem ersten Speicher 22 gespeicherten Vergleichswerten vergleicht und ein Tachykardiegefahrensignal ausgibt, falls der Vergleich ergeben sollte, daß die von der Meßwertverarbeitungseinheit übermittelten Werte für die Parameter $\tau_x$ und g in die Gefahrenzone bzw. den Tachykardiegefahrenbereich fallen.

In dem zweiten Speicher 24 sind Wertepaare für zu früheren Meßzeitpunkten abgeleitete Werte der Parameter $\tau_x$ und g abgespeichert. Durch Vergleich der jeweils jüngst abgeleiteten Werte für die Parameter $\tau_x$ und g mit denjenigen vorangegangener Meßzeitpunkte bestimmt die Auswerteeinheit 20 einen Trend für zukünftige Werte der Parameter $\tau_x$ und g. Weist dieser Trend in Richtung solcher Werte von $\tau_x$ und g, die in der Gefahrenzone bzw. dem Tachykardiegefahrenbereich liegen, gibt die Auswerteeinheit 20 auch dann ein Tachykardiegefahrensignal aus, wenn die zuletzt von der Meßwertverarbeitungseinheit übermittelten Werte dieser Parameter nicht in der Gefahrenzone liegen. Alternativ ermittelt die Auswerteeinheit 20 aus den zuletzt von der Meßwertverarbeitungseinheit übermittelten Werten für $\tau_x$ und g sowie den in dem zweiten Speicher 24 gespeicherten Werten vorangegangener Meßzeitpunkte für die Zukunft extrapolierte Werte für die Parameter $\tau_x$ und g. Liegen diese extrapolierten Parameterwerte in dem Tachykardiegefahrenbereich gibt die Auswerteeinheit 20 ebenfalls ein Tachykardiegefahrensignal aus.

Das Tachykardiegefahrensignal wird an eine Elektrostimulationseinheit 26 weitergeleitet, die dazu mit der Auswerteeinheit 20 verbunden ist. Die Elektrostimulationseinheit 26 kann ein Elektrostimulator, wie beispielsweise ein Kardioverter oder ein Defibrillator, sein. Die Elektrostimulationseinheit 26 ist über eine elektrische Leitung mit elektrisch leitenden Oberflächenbereichen der in das Herz 14 eingeführten Elektrodenleitung 12 verbunden, um über die elektrisch leitenden Oberflächenbereiche auf der Elektrodenleitung 12 in an sich bekannter Weise Stimulationsimpulse an das Herz 14 abzugeben und auf diese Weise eine Tachykardie zu bekämpfen.

Die Elektrodenleitung 12 kann dabei in an sich bekannter Weise so an die Vorrichtung 10 angeschlossen sein, daß die zu den leitenden Oberflächenbereichen, die beispiels, weise als Ring- oder Tipelektrode ausgebildet sein können, führenden Leitungen umschaltbar sind, so daß die elektrisch leitenden Oberflächenbereiche einerseits zum Abgeben von Stimulationsimpulsen an das Herz 14 und andererseits zur Aufnahme elektrischer Signale vom Herzen 14 umgeschaltet werden können. In einem Schaltzustand sind die elektrisch leitenden Oberflächenbereiche mit der Meßeinheit 16 verbunden, um dieser elektrische Signale des Herzens zuzuführen. In einem anderen Schaltzustand sind die elektrisch leitenden Oberflächenbereiche mit der Elektrostimulationseinheit 26 verbunden, um elektrische Stimulationsimpulse an das Herz 14 abzugeben.

Zum tieferen Verständnis der Erfindung dienen die folgenden Ausführungen, denen insbesondere die Ableitung der Parameter $\tau_x$ und g aus Meßwerten für die Herzrate und die Aktionspotentialdauer zu entnehmen sind sowie Ausführungen zur Bestimmung eines Trends dieser Parameter und Angaben zu einem Tachykardiegefahrenbereich, der in den folgenden Ausführungen als Gefahrenzone bezeichnet ist.

## 1 Einleitung

Mit der Fragestellung nach frühen Anzeichen einer sich anbahnenden Arrhythmie kristallisieren sich im wesentlichen zwei Aspekte heraus, die zum einen die zeitliche Koordination der Myokarderregung und zum anderen die Bereitschaft des Substrates für eine Arrhythmie betreffen.

Von den in [[12]] bereits skizzierten Möglichkeiten zur Früherkennung von Herzrhythmusstörungen vertieft diese Arbeit den zweiten Teilaspekt, nämlich die Frage, ob und wie aus der Systembeobachtung Rückschlüsse auf elektrophysiologische Veränderung im Herzmuskelgewebe gezogen werden können. Es ist bekannt, daß selbst im Gesunden zufällig entstandene, sogenannte ektopische Herzschläge zu beobachten sind. Andererseits aber zeigen klinische Studien, daß bei entsprechenden Herzerkrankungen selbst schon ein einziger in einem kritischen Zeitfenster eingebrachter Stimulus eine Arrhythmie auslösen kann [[3]]. Die Ursache, daß der ektopische Herzschlag im gesunden Myokard keine Arrhythmie auslöst, ist auf die unterschiedlichen funktionellen Eigenschaften des gesunden bzw. erkrankten Syncytiums zurückzuführen.

Eine weitere Überlegung frühe Anzeichen einer sich anbahnenden Arrhythmie durch Analyse der Substrateigenschaften zu identifizieren, wird dadurch bestärkt, daß erwartungsgemäß Veränderungen im Substrat die Folge eines längerfristigen Prozesses sind. Es ist also davon auszugehen, daß durch diesen Ansatz länger schon vor dem Eintreten der Herzrhythmusstörungen diese Gefahr erkannt und damit rechtzeitig therapiert werden kann.

## 2 Voraussetzungen für die Systemidentifikation

Gegenstand der Betrachtungen ist das myokardiale Syncytium, das sich als ein nichtlineares dynamisches System manifestiert. Ziel ist es, basierend auf Methoden der Systemidentifikation Parameter zu definieren und quantitativ zu bestimmen, die Arrhythmie-relevante Eigenschaften des Syncytiums charakterisieren. Zur Durchführung der Systemidentifikation müssen Ein- und Ausgangssignal bekannt sein, wobei das Eingangssignal das System auslenken, d.h. die internen Zustandsgrößen anregen muß. Die konkrete Wahl von Ein- und Ausgangssignal erfolgt aufgrund physiologischer Kriterien, die an späterer Stelle diskutiert werden. Sofern das System im systemtheoretischen Sinne beobachtbar ist, können damit aus dem Übertragungsverhalten die Systemparameter ermittelt werden. Im konkreten Fall kann diese Forderung etwas eingeschränkt werden, insoweit, daß das System (nur) hinsichtlich der Arrhythmie-relevanten Paramter beobachtbar sein muß.

Die Festlegung dieser Parameter ist vom Modellansatz für das zu beobachtende System abhängig. In der Praxis wird meist mangels näherer Informationen über das System eine "Black Box"-Beschreibung gewählt, wobei dann lediglich die Ordnung des Systems festzulegen ist. Ist das System zusätzlich linear, können etablierte Verfahren der Systemidentifikation aus der Literatur [[9]] herangezogen werden. Da das myokardiale Syncytium jedoch ein nichtlineares System ist, wurde in [[12]] ein Ansatz vorgestellt, der basierend auf Voruntersuchungen aus [[6]] eine Beschreibung des betrachteten Systems als Kettenschaltung eines linearen dynamischen und eines nichtlinearen statischen Teilsystems ermöglicht. Zur Identifikation des linearen Anteils, dem in [[12]] eine "Black Box"-Beschreibung zugrunde liegt, kann dann ein Standardverfahren eingesetzt werden. Der Nachteil des "Black Box"-Ansatzes besteht jedoch darin, daß den daraus resultierenden Parametern keine unmittelbare physiologische Bedeutung zugeordnet werden kann. Die Kriterien und Schwellwerte für die Arrhythmieprädiktion müssen folglich anhand von zahlreichen klinischen Studien und statistischen Analysen hergeleitet werden.

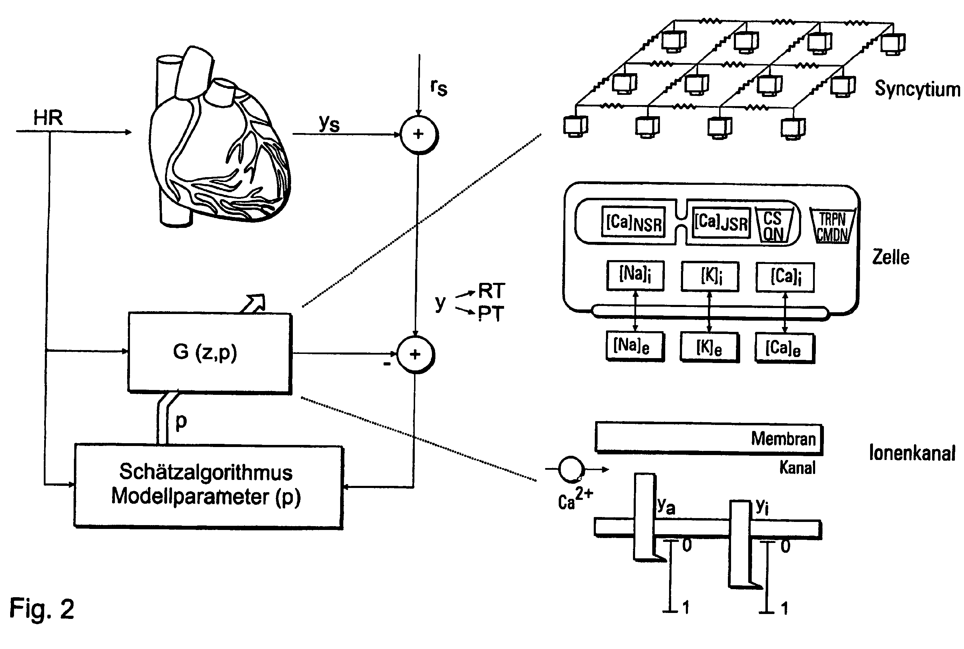

Deshalb baut der in dieser Arbeit vorgestellte erweiterte Ansatz auf einem physiologisch begründeten Modell des Substrats auf. Dabei wird die Modellbildung dahingehend gelenkt, daß insbesondere die aus physiologischer Sicht Arrhythmie-relevanten Parameter herausgearbeitet werden. Ausgangspunkt ist ein mathematisches Modell der Herzmuskelzelle, die einerseits als Netzwerkknoten im Muskelgewebe zu betrachten ist und andererseits über die dynamisch agierenden Ionenkanäle den Ionenfluß zwischen Intra- und Extrazellulärraum ermöglicht (Fig 2).

Im weiteren ist nun festzulegen, welche konkreten Meßgrößen als Ein- und

Ausgangssignal für die Systemidentifikation zu wählen sind. Basierend auf der in [[12]] durchgeführten Literaturstudie erwiesen sich zwei fundamentale dynamische Abhängigkeiten physiologischer Größen als aussagekräftig für eine Systemidentifikation im Hinblick auf die Früherkennung von Arrhythmien, nämlich:

• der Zusammenhang zwischen Herzrate (HR) und Repolarisationszeit (repolarization time, RT) und

• der Zusammenhang zwischen Herzrate und Ausbreitungsgeschwindigkeit der Erregung. Gemessen wird die Erregungslaufzeit (propagation time, PT) zwischen 2 definieren Meßpunkten.

Die vorliegende Arbeit vertieft zunächst den ersten Aspekt, also die Frage welche Gesetzmäßigkeit zwischen der Herzrate (bzw. der Zykluslänge = Herzschlagperiode) und der Repolarisationszeit besteht, sowie die Frage welche physiologischen Parameter diesen Zusammenhang im Falle pathologischer Substratveränderungen beeinflussen.

Es sei darauf hingewiesen, daß unter HR genauer gesagt die lokale Herzrate zu verstehen ist, also die Ereignisrate am Meßort (deswegen auch als event rate, ER differenziert). Offensichtlich ist bei einer bereits unkoordinierten Erregungsausbreitung der Begriff Herzrate streng genommen gar nicht definiert und ER je nach Meßort unterschiedlich. Die zentrale Aufgabe besteht jedoch darin, ein Verfahren zu entwickeln, das bereits in einem frühen Stadium der Erkrankung auf die drohende Arrhythmiegefahr hinweist, also dann schon, wenn der Herzmuskel noch weitestgehend normal funktioniert. Unter diesen Randbedingungen läuft die Erregung koordiniert ab und ER stimmt unabhängig vom Meßort mit HR überein. Aus diesem Grund, und auch um die übliche Terminologie zu verwenden, wird im folgenden stets die Größe HR verwendet.

Die Repolarisationszeit stellt das Zeitintervall zwischen dem Erregungszeitpunkt der Zelle und dem Zeitpunkt der Rückkehr zum Ruhemembranpotential dar, d.h. die Aktionspotentialdauer (APD, hier stellvertretend für $APD_{90}$). Streng gesehen ist sie allerdings ein Parameter der die einzelne Zelle charakterisiert.

Die unter diesem Gesichtspunkt zu beobachtenden Größen für die Systemidentifikation sind damit:

• HR - als Eingangssignal und

• RT - als Ausgangssignal.

Die Bestimmung von HR (als "lokale Herzrate") ist durch die heute verfügbare Technik eine bereits gelöste Aufgabe. Andererseits ist die Korrelation zwischen Aktionspotentialdauer und beispielsweise dem QT-Intervall des EKGs sowohl experimentell als auch theoretisch in guter Näherung nachgewiesen. Ähnliche Überlegungen gelten auch für das IEGM, XAP oder VER, so daß für die meßtechnische Bestimmung eines Näherungswertes der Repolarisatiottszeit mehrere Alternativen zur Verfügung stehen. Welche davon die günstigste ist, muß noch anhand von praktischen Gesichtspunkton der klinischen Umsetzung sowie dem rechentechnischen Aufwand der Signalverarbeitung entschieden werden. Es ist jedoch festzuhalten, daß sowohl das Eingangssignal als auch das Ausgangssignal als notwendige Voraussetzungen für die Systemidentifikation unter vertretbarem technischen Aufwand gemessen werden können.

**3 Klinischer Hintergrund**

Der physiologische Informationsgehalt des dynamischen Zusammenhangs zwischen HR und RT (bzw. APD) ist in der Literatur vielfach untersucht worden und zwar insbesondere auch im Hinblick auf frühe Anzeichen der Entstehung von Herzrhythmusstörungen. Dieser Fragestellung wurde sowohl unter klinischen als auch elektrophysiologischen Aspekten nachgegangen, wobei sich zwei Meßprotokolle etabliert haben, die den Zusammenhang zwischen HR und APD in Kennlinienform charakterisieren.

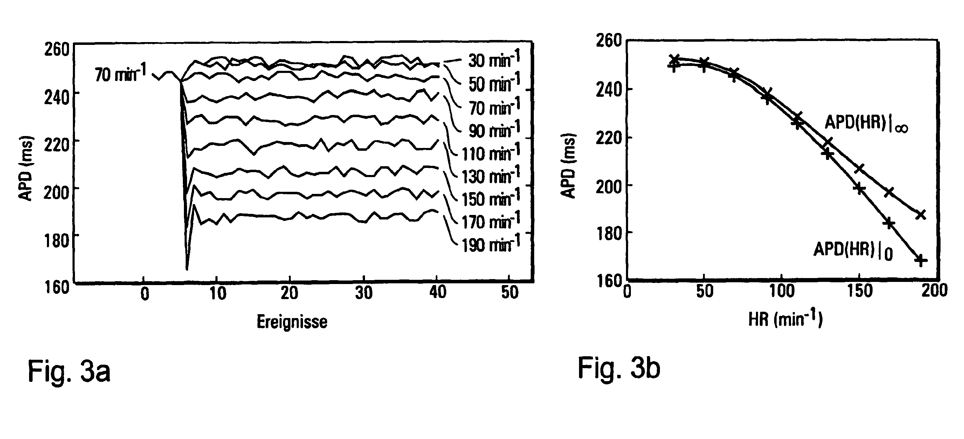

• ***Bestimmung der*** $APD(HR)|_0$ ***-Kennlinie ("restitution curve")***
Das Substrat wird zunächst eine Zeit lang mit konstanter Frequenz stimuliert und ausgehend davon ein Frequenzsprung vorgenommen. Die restitution curve resultiert, wenn die Repolarisationszeit des ersten Herzschlags nach dem Frequenzsprung über der entsprechenden (neuen) Stimulationsrate aufgetragen wird.
• ***Bestimmung der*** $APD(HR)|_\infty$ ***-Kennlinie (im eingeschwungenen Zustand)***
Die Repolarisationszeit wird ebenfalls für verschieden Stimulationsfrequenzen gemessen, jedoch erst nach dem ein "eingeschwungener Zustand" erreicht wurde. Wie aus Fig. 3a hervorgeht, weist das System offensichtlich einen internen Grenzzyklus auf. Daher wird als eingeschwungener Wert der Mittelwert über mehrere Zyklen genommen und über der entsprechenden Stimulationsrate aufgetragen.

Betrachtet man Fig. 3a, die den gesamten Einschwingprozeß infolge des Frequenzsprungs darstellt, so erhält man die restitution curve, wenn die Spitzenwerte unmittelbar nach den Sprung verbunden werden. Hingegen resultiert die $APD(HR)|_\infty$ -Kennlinie, wenn man die Mittelwerte der APD-Zeiten zu einem reichlich späteren Zeitpunkt aufträgt.

Die wichtigsten Beobachtungen und Schlußfolgerung der Literatur im Zusammenhang mit der Bestimmung dieser Kennlinien und deren Abhängigkeit von krankhaften bzw. kontrolliert veränderten Substrateigenschaften können wie folgt zusammengefaßt werden:

- Untersuchungen des elektrischen Remodellierungsphänomens am menschlichen Atrium haben gezeigt, daß sich im Vorfeld von AF sowohl die restitution curve als auch die $APD(HR)|_\infty$ -Kennlinie verändern und zwar sich einerseits verschieben und andererseits flacher werden [[2]].

- Im Falle Ischämie-betroffener Bereiche im menschlichen Endokard war ebenfalls eine Abflachung der restitution curve nachweisbar [[8]], wobei hieraus Rückschlüsse für die Arrhythmogenese abgeleitet wurden. Auf einen allgemeinen Zusammenhang zwischen den frequenzabhängigen elektrophysiologischen Eigenschaften des myokardialen Syncytiums in Form der restitution curve und der Entstehung von Herzrhythmusstörungen wird auch in [[4]] hingewiesen.

- Besonders aufschlußreich für die Festlegung der Arrhythmie-relevanten Parameter sind zwei weitere elektrophysiologische Studien. Zum einen wurde basierend auf Messungen des zellulären Aktionspotentials der Einfluß von Sympathikomimetika (β-adrenerge Stimulation durch den Ca-Kanal-Agonisten Dobutamin) untersucht [[5]] und daraus geschlußfolgert, daß die beobachteten Veränderungen der restitution curve mit einer Erhöhung der Ca-Kanal-Leitfähigkeit zusammenhängen. Zum anderen wird gezielt nachgewiesen [[7]], daß Veränderungen in der restitution curve auf Eigenschaften der K- und wiederum der Ca-Kanäle zurückzuführen sind. Um diese Mechanismen selektiv zu untersuchen, wurden dabei spezifische Kanalblocker eingesetzt.

Basierend auf diesen Erkenntnissen bleibt nun zu klären, welcher physiologisch begründbare Modellansatz die dynamische Abhängigkeit zwischen HR und APD (bzw. RT allgemein) erklärt und über welche Mechanismen bestimmte zelluläre Parameter diese Gesetzmäßigkeit beeinflussen. Ziel ist dabei, diese Parameterwerte mittels eines Systemidentifikationsansatzes zu ermitteln, um dann die Arrhythmiegefahr aufgrund von bekannten elektrophysiologischen Kriterien beurteilen zu können.

## 4 Mathematischer Zusammenhang zwischen Repolarisationszeit und Herzrate

Ausgehend von der mathematischen Modellbeschreibung der Herzmuskelzelle wird im folgenden der Zusammenhang zwischen Repolarisationszeit und Herzrate hergeleitet. Ziel ist dabei, die meßtechnisch bestimmbare $APD(HR)|_\infty$ -Kennlinie formelmäßig durch einen geschlossenen Ausdruck zu beschreiben, um dann aus den Meßdaten auf die physiologischen Parameter rückrechnen zu können, die diese Gesetzmäßigkeit beeinflussen.

### 4.1 Qualitative Beschreibung des Phänomens

Sowohl aus experimentellen Studien als auch aus Modellrechnungen ist bekannt, daß sich die Aktionspotentialdauer verkürzt, wenn die Herzrate ansteigt bzw. wenn die Zykluslängen verringert wird, und umgekehrt. Die Ursachen dieses Mechanismus zu klären, stellt die Schlüsselfrage für die Herleitung der APD(HR)-Kennlinien dar.

Betrachtet man hierzu die Zustandsgrößen des Zellmodells [[6]], so kann festgestellt werden, daß sich diese teils mit sehr kurzen, teils mit längeren Zeitkonstanten verändern. Dieser Tatsache zufolge verkörpert die Zelle ein steifes dynamischen System. Während die Zeitkonstanten der schnellen Zustandsgrößen im Vergleich zur APD verschwindend klein sind, erreichen die Torvariablen f und insbesondere x selbst lange nach Rückkehr des Aktionspotentials zur Ruhemembranspannung noch nicht ihren eingeschwungenen Zustand. Die Variable f beschreibt dabei den Öffnungszustand des Inaktivierungstors des Ca-Kanals und x den Öffnungszustand des dynamischen K-Kanals. Beide Variablen variieren zwischen 0 und 1 (relativer Öffnungszustand).

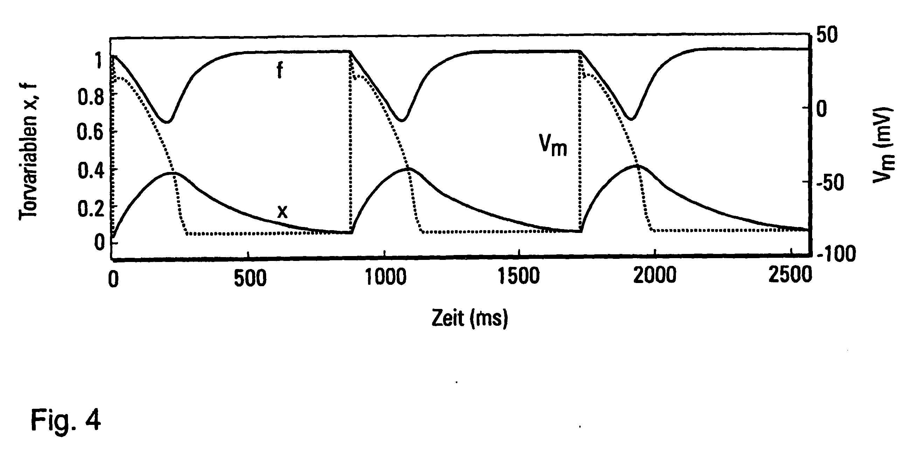

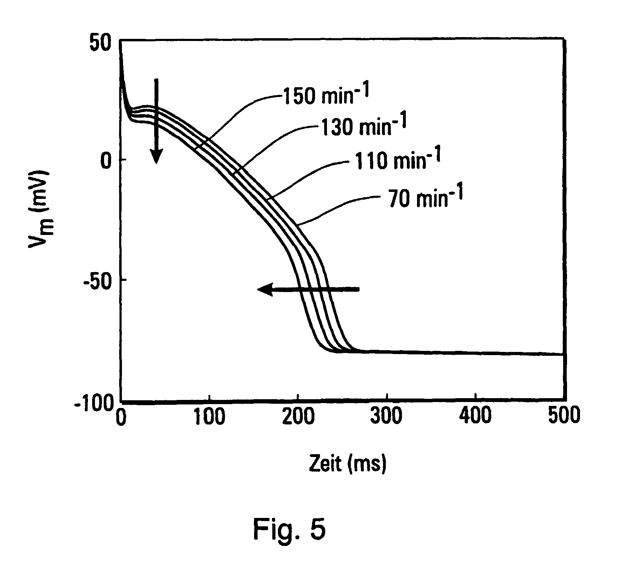

Bei einer verkürzten Zykluslänge setzt der neue Stimulus entsprechend früher ein, so daß sich die Anfangswerte der Zustandsvariablen f und x für den neuen Zyklus signifikant von denen des vorherigen Zyklus unterscheiden. Es ist daher offensichtlich, daß entsprechende Veränderungen der charakteristischen Abmessungen des Aktionspotentials zu erwarten sind. Konkret ist der Anfangswert von x in diesem Beispielsfall größer (Fig. 4), d.h. der K-Strom startet bereits von einem erhöhten Wert. Damit wird das labile Gleichgewicht der Ionenströme während der Plateauphase früher in Richtung eines repolarisierenden Zellauswärtsstroms gekippt; die Aktionspotentialdauer verkürzt sich. Ähnliche Überlegungen lassen sich für den Ca-Kanal anstellen. Bei verkürzter Stimulationsperiode ist die f-Variable noch spürbar kleiner als 1, also der Ca-Kanal noch nicht voll aktivierbar. So wird im nächsten Zyklus der Ca-Strom geringer ausfallen, was einerseits zu einer Abnahme der Plateauhöhe führt. Andererseits überwiegt dann der repolarisierende K-Strom schon zu einem früheren Zeitpunkt, so daß ebenfalls eine Verkürzung des Aktionspotentials die Folge ist. Fig. 5 veranschaulicht diese Effekte anhand einer Simulationsrechnung.

Im folgenden soll nun die Abhängigkeit der Aktionspotentialdauer von der Stimulationsrate mathematisch ausgedrückt werden, wobei letztendlich ein analytischer Ausdruck angestrebt wird, der diese beiden Größen verbindet. Berücksichtigt man jedoch die sehr umfangreiche Beschreibung sowie die hochgradige Nichtlinearität der Modellgleichungen [[6]], scheitert dieses Vorhaben, da eine geschlossene analytische Lösung des Differentialgleichungsystems nicht möglich ist. Es ist daher notwendig aufgrund des Modellverständnisses das oben beschriebene Phänomen in einer vereinfachten mathematischen Form darzustellen.

### 4.2 Quantitativer Modellansatz

Wie bereits qualitativ beschrieben, ist die Ursache für die APD(HR)-Abhängigkeit (im wesentlichen) allein auf die Zustandsvariablen f und x zurückzuführen, so daß auch nur diese im Modellansatz berücksichtigt werden sollen. Gesucht ist demnach der dynamische Zusammenhang zwischen diesen Zustandsgrößen und der Repolarisationszeit.

Aufgrund des Hodgkin-Huxley-Ansatzes [[1]] wird das dynamische Verhalten einer solchen Torvariable (stellvertretend mit y bezeichnet) durch das folgende Anfangswertproblem beschrieben:

$$\begin{cases} \dfrac{dy}{dt} = \dfrac{y_\infty - y}{\tau_y}; & t \geq t_v \\ y(t_v) = y_v \end{cases} \qquad (1)$$

Dabei wird jedoch vereinfachend angenommen, daß das Relaxationsverhalten dieser Variable hier nicht von dem aktuellen Wert der Transmembranspannung abhängen soll, sondern durch eine feste Zeitkonstante $\tau_y$ in adäquater Höhe charakterisiert wird. Was das Aktionspotential selbst betrifft, wird lediglich zwischen einem erregten und einem unerregten Zustand unterschieden, so daß die konkrete Form des Aktionspotential nebensächlich ist. Für diese vereinfachten Modellbetrachtungen wird damit der Verlauf der Transmembranspannung $V_m$ durch ein Rechtecksignal genähert, dessen Amplitude und Dauer denen des Aktionspotentials entsprechen:

$$V_m(\hat{t}) \approx V_{m0} + A_v \cdot \left[ s(t_n) - s(t_n - T_n) \right] \qquad (2)$$

$V_{m0}$ und $A_v$ bezeichnen die Ruhemembranspannung und Amplitude des Aktionspotentials, $s(t)$ ist die Heaviside'sche Sprungfunktion.

Damit nimmt $y_\infty$ nur noch 2 Werte an, nämlich $y_s$ während der Systole (erregter Zustand) und $y_d$ während der Diastole (unerregter Zustand), so daß die Differentialgleichung aus (1) innerhalb eines jeden dieser beiden Zeitintervalle linear wird. Bereichsweise hat dann y(t) einen exponentiellen Verlauf.

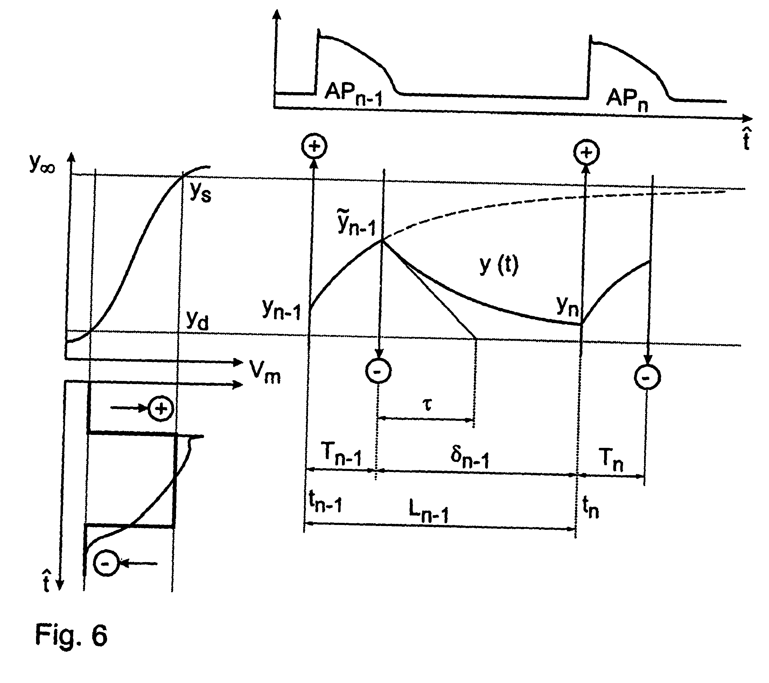

Diese dem Modellansatz zugrunde liegenden Überlegungen sind in Fig. 6 skizziert. Dabei stellt y beispielhaft eine Näherung der Torvariable x dar (man beachte die Übereinstimmung mit Fig. 4).

Im konkreten Fall der Zelle wird die Repolarisationszeit durch die Aktionspotentialdauer dargestellt, die in den Rechnungen einfachheitshalber mit T bezeichnet wird. Für die Herleitung ist es günstiger, statt der Herzrate die reziproke Größe, also die Zykluslänge L (Stimulationsperiode) herzunehmen. Die Dauer der Diastole wird mit δ bezeichnet. Bezeichnet der Index dieser Größen die Nummer des jeweiligen Aktionspotentials, so gilt:

$$L_v = T_v + \delta_v \qquad (3)$$

Aus der Lösung des Anfangswertproblems (1) ergibt sich dann für die Randpunkte des systoloschen ($T_{n-1}$) und diastolischen ($\delta_{n-1}$) Intervalls (siehe Fig. 6):

$$\begin{cases} \tilde{y}_{n-1} = (y_{n-1} - y_s)e^{-\frac{T_{n-1}}{\tau}} + y_s \\ y_n = (\tilde{y}_{n-1} - y_d)e^{-\frac{\delta_{n-1}}{\tau}} + y_d \end{cases} \tag{4}$$

Damit stehen die y-Anfangswerte zweier aufeinanderfolgender Zyklen (n-1 und n) in folgendem Zusammenhang:

$$y_n = (y_{n-1} - y_s)e^{-\frac{L_{n-1}}{\tau}} + (y_s - y_d)e^{-\frac{L_{n-1}-T_{n-1}}{\tau}} + y_d \tag{5}$$

Unter Verwendung von (5) resultieren für die Torvariablen x und f die folgenden (genäherten) Beziehungen:

$$x_n = (x_{n-1} - x_s)e^{-\frac{L_{n-1}}{\tau_x}} + (x_s - x_d)e^{-\frac{L_{n-1}-T_{n-1}}{\tau_x}} + x_d$$

$$f_n = (f_{n-1} - f_s)e^{-\frac{L_{n-1}}{\tau_f}} + (f_s - f_d)e^{-\frac{L_{n-1}-T_{n-1}}{\tau_f}} + f_d \tag{6 a, b}$$

Als nächstes muß nun der Bezug dieser Zustandsvariablen zur Aktionspotentialdauer hergeleitet werden. Dazu ist es hilfreich, den zeitlichen Verlauf der wichtigsten Ionenströme des Zellmodells zu analysieren, um ein mathematisches Kriterium für das Auslösen der Repolarisation im Aktionspotential zu definieren.

Die im Bezug auf die Repolarisation relevanten Ionenströme sind der Ca-Strom, und die Ströme durch den dynamischen sowie den statischen K-Kanal, deren Summenstrom während der Repolarisationsphase zellauswärts gerichtet ist. Sie gehen in Form der Stromdichten $J_{Ca}$, $J_{Kx}$ und $J_{Kl}$ in die Rechnung ein. Der Na-Strom hingegen ist hier vernachlässigbar, da er außer während der Depolarisation nahezu 0 ist.

Während der Repolarisation ist der resultierende Membranstrom zellauswärts gerichtet; es gilt dann:

$$J_{Ca} + J_{K1} + J_{Kx} > 0 \tag{7}$$

Insbesondere unter der Annahme, daß das Aktionspotential hier als Rechteckimpuls approximiert wird (Gleichung (2)), sind die Stromdichten $J_{Ca}$ und $J_{Kl}$ (d.h. auch ihre Summe) während der Plateauphase näherungsweise konstant. Der Wert dieser Konstanten hängt allerdings im Falle des Ca-Kanals noch vom Öffnungszustand des Inaktivierungstors (f) zu Beginn des Aktionspotential ab. Die in [[6]] verwendeten allgemeinen Ansätze vereinfachen sich daher zu:

$$J_{Ca} \approx -f_n A_{Ca}$$

$$J_{K1} \approx A_{K1}$$

$$J_{Kx} \approx x^2(t) A_{Kx} \tag{8 a, b, c}$$

Allein $J_{Kx}$ ist dann während der Plateauphase noch zeitlich veränderlich und bestimmt daher wann sich die Stromrichtung ändert. Der Geltungsbereich dieser Beschreibung (8) beschränkt sich auf die Plateauphase (Diastole). Daß aufgrund dieser Vereinfachten Darstellung der Ionenströme der Summenstrom und damit $dV_m/dt$ während der Plateauphase ungleich 0 ist, d.h. das Aktionspotentialplateau streng gesehen nicht eben sein kann (wie durch Gleichung (2) beschrieben), muß als Folge dieser Vereinfachungen in Kauf genommen werden. Letztendlich ist hier die Dauer (Pulsbreite) des Aktionspotential von Interesse und nicht die genaue Signalmorphologie.

Als Repolarisationszeitpunkt soll hier der Zeitpunkt des Stromgleichgewichtes betrachtet werden, der durch

die Gleichung:

$$\left(J_{Ca} + J_{K1} + J_{Kx}\right)\Big|_{t=T_n} = 0 \tag{9}$$

definiert ist. Setzt man nun die vereinfachten Ausdrücke für die Ionenströme aus (8) in die Repolarisationsbedingung (9) ein, resultiert die Gleichung:

$$x^2(T_n) - c_1 f_n + c_0 = 0$$

mit

$$c_1 = \frac{A_{Ca}}{A_{Kx}}; \quad c_0 = \frac{A_{K1}}{A_{Kx}} \tag{10 a, b, c}$$

die bezüglich $T_n$, der Aktionspotentialdauer des betrachteten Zyklus n, zu lösen ist.

Die zeitveränderliche Größe x(t) ist Lösung einer Gleichung der Form (1), die unter den genannten Rahmenbedingungen im Geltungsbereich von (8) die Gestalt einer linearen Differentialgleichung erster Ordnung annimmt. Damit ist:

$$x(t) = \left(x_n - x_s\right)e^{-\frac{t}{\tau_x}} + x_s \quad \text{für} \quad 0 \leq t \leq T_n; \quad t = \hat{t} - t_n$$

und

$$\tag{11 a, b}$$

$$x^2(t) = \left(x_n - x_s\right)^2 e^{-\frac{2t}{\tau_x}} + 2x_s\left(x_n - x_s\right)e^{-\frac{t}{\tau_x}} + x_s^2$$

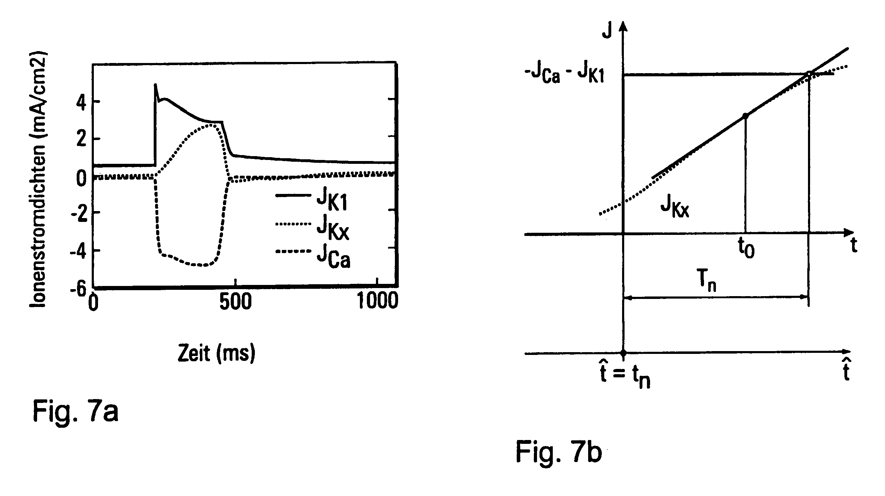

Aus Fig. 7a und 7b ist zu erkennen, daß $J_{Kx}$ im betrachteten Zeitintervall der Systole, in guter Näherung linear verläuft. Es ist daher zur Vereinfachung der weiteren Rechenschritte zulässig, eine Linearisierung des Ansatzes für $J_{Kx}$ in einem Punkt $t=t_0$ vorzunehmen. Mit:

$$x^2(t) \approx \left(x_n - x_s\right)^2 \eta^2 + 2x_s\left(x_n - x_s\right)\eta + x_s^2 - \frac{2\eta}{\tau_x}\left[\left(x_n - x_s\right)^2 \eta + x_s\left(x_n - x_s\right)\right]\left(t - t_0\right)$$

folgt

$$x^2(t) \approx \left(a_2 x_n^2 + a_1 x_n + a_0\right)\frac{t}{\tau_x} + \left(b_2 x_n^2 + b_1 x_n + b_0\right) \tag{12}$$

wobei

$$\eta = e^{-\theta_0}; \quad \theta_0 = \frac{t_0}{\tau_x}$$

$$\begin{cases} a_2 = -2\eta^2 \\ a_1 = -2x_s\eta(1-2\eta) \\ a_0 = 2x_s^2\eta(1-\eta) \end{cases} \qquad \begin{cases} b_2 = \eta^2(1+2\theta_0) \\ b_1 = 2x_s\eta(1-\eta) + 2\theta_0 x_s\eta(1-2\eta) \\ b_0 = x_s^2(1-\eta)^2 - 2\theta_0 x_s^2\eta(1-\eta) \end{cases}$$

gilt. Wählt man $t_0 = \tau_x/2$ (z.B. $t_0 = 150$ ms), so liegt der Linearisierungspunkt unter physiologisch zu erwartenden Rahmenbedingungen mit Sicherheit innerhalb der Plateauphase des Aktionspotentials. Gängige Werte der Aktionspotentialdauer (T) liegen etwa zwischen 100 ms und 400 ms (häufig $T \approx 200$ ms), während $\tau_x$ im erregten Zustand der Zelle Werte zwischen 100 ms und 300 ms annehmen kann. Unter den vereinfachenden Annahmen ist $\tau_x$ in der Herleitung jedoch als Konstante anzusehen, deren Wert innerhalb dieses möglichen Bereichs liegt. Diese Annahmen bezüglich T und $\tau_x$ gelten zwar nur solange eine noch weitestgehend normale Funktionalität der Zelle vorausgesetzt werden kann. Davon ist jedoch auszugehen, da das zu entwickelnde Verfahren ohnehin dem Anspruch genügen soll, Hinweise einer möglichen Erkrankung zu einem Zeitpunkt zu liefern, zu dem die Zelle zumindest anscheinend noch intakt ist.

Unter Anwendung der Näherungsformel (12), die in (10 a) eingesetzt wird, erhält man zusammen mit (6 a, b) die Beziehungen:

$$\begin{cases} T_n = \tau_x \dfrac{c_1 f_n - c_0 - \left(b_2 x_n^2 + b_1 x_n + b_0\right)}{a_2 x_n^2 + a_1 x_n + a_0} \\[2em] x_n = \left(x_{n-1} - x_s\right)e^{-\frac{L_{n-1}}{\tau_x}} + \left(x_s - x_d\right)e^{-\frac{L_{n-1}-T_{n-1}}{\tau_x}} + x_d \\[2em] f_n = \left(f_{n-1} - f_s\right)e^{-\frac{L_{n-1}}{\tau_f}} + \left(f_s - f_d\right)e^{-\frac{L_{n-1}-T_{n-1}}{\tau_f}} + f_d \end{cases} \qquad (13\ a, b, c)$$

die ausgehend von den Anfangswerten

$$\left(T_0, \quad x_0, \quad f_0\right) \qquad\qquad\qquad\qquad (13\ d)$$

das Systemverhalten hinsichtlich der interessierenden Eigenschaften (zwar unter vereinfachenden Annahmen) vollständig beschreiben.

Diese mathematische Modelldarstellung stellt ein wichtiges Teilergebnis für die angestrebte Systemidentifikation dar, deren Gleichungen den nichtlinearen dynamischen Zusammenhang zwischen den Zustandsgrößen: Aktionspotentialdauer (T) und den Torvariablen x und f zum Ausdruck bringen. Eingangssignal des Systems ist dabei die Zykluslänge L. Als wichtigste Größen bezüglich der Früherkennung von Arrhythmien sind die Modellparameter $\tau_x$, $c_1$ und $c_0$ hervorzuheben. $\tau_x$ stellt dabei die Zeitkonstante des dynamischen K-Kanals dar, während $c_1$ und $c_0$ gemäß ihrer Definition in (10 b, c) die Verhältnisse der Ca- und K-Stromamplituden ausdrücken. Letztendlich sind letztere ein Maß für die Leitfähigkeiten der betreffenden Ionenkanäle und liefern Aussagen über deren Arbeitspunkte.

Im Gegensatz zu dem in [[6]] oder anderen Arbeiten der Literatur eingesetzten Zellmodellen steht die in (13) zusammengefaßte Systembeschreibung für ein diskretes Ersatzmodell der Zelle im Hinblick auf die Abhängigkeit der Aktionspotentialdauer von der Stimulationsfrequenz. Die sonst kontinuierlichen Zustandsgrößen x und f werden hier durch die Zahlenfolgen $x_n$ und $f_n$ repräsentiert, die jeweils die Anfangswerte der betreffenden kontinuierlichen Größen zu Beginn eines jeden Aktionspotenials darstellen.

Unter Kenntnis der physiologischen Modellparameter und des Zustandsvektors entsprechend eines Aktionspotentials ist es nun möglich die Dauer des nächstfolgenden Aktionspotentials zu berechnen. Diese rekursive Rechenvorschrift bringt auch den Nachweis des in der Literatur oft diskutierten 'memory'-Effekts der Herzmuskelzelle. Wie weit jedoch das Gedächtnis meßbar in die Vergangenheit reicht, hängt von den konkreten Systemparametern ab.

### 4.3 Herleitung der APD(HR)-Kennlinie im eingeschwungenen Zustand

Obwohl nun eine vollständige Systembeschreibung vorliegt, ist die Rückrechnung der Zellparameter, die Aufschlüsse über den arrhythmogenen Zustand des Substrats liefern sollen, in dieser Form noch nicht möglich. Bezüglich der hier vorliegenden Fragestellung sind unter klinisch vertretbarem Aufwand nur die Zykluslänge L und die Repolarisationszeit T (in Form der APD, der QT-Zeit oder ähnlicher Größen) meßbar. Die Modellgleichungen (13) beinhaltet jedoch noch innere Größen (Torvariablen x und f), die meßtechnisch nicht zugänglich sind. Das Ziel der nächsten Maßnahmen besteht nun darin, diese inneren Größen zu eliminieren.

Dazu wird das System in einem eingeschwungenen Zustand betrachtet, also dann, wenn dieses bereits eine längere Zeit mit einer konstanten Frequenz stimuliert wurde, so daß transiente Vorgänge abgeschlossen sind. Aus Fig. 3a ist zwar ersichtlich, daß die Zelle bezüglich der Aktionspotentialdauer einen inneren Grenzzyklus aufweist; dieser soll allerdings bei der Herleitung vernachlässigt werden. Da ohnehin an das Verfahren der Anspruch gestellt wird, Hinweise auf eine sich anbahnende Arrhythmie bereits dann zu liefern, wenn noch kein signifikanter Anstieg in der Herzrate ersichtlich ist, kann auf die Torvariable f als Zustandsvariable verzichtet werden. Ist die Zykluslänge nämlich ausreichend groß (Stimulationsfrequenz entsprechend klein) so ist f zu Beginn eines jeden Aktionspotential bereits eingeschwungen und hat etwa den Wert $f_n = 1$. Ferner ist aus Untersuchungen an Zellmodellen bekannt, daß der diastolische asymptotische Wert der x-Variable ($x_d$) annähernd 0 ist und $x_n$ als Anfangswert von x zu Beginn des Aktionspotentials (insbesondere bei nicht all zu hohen Stimulationsraten) wesentlich kleiner als 1 ist. Die Vereinfachungen die sich hieraus ergeben, werden mathematisch wie folgt formuliert:

- Grenzzyklen vernachlässigt (eingeschwungener Zustand)

$$\Rightarrow \begin{cases} x_n \approx x_{n-1} = \hat{x} \\ T_n \approx T_{n-1} = T \quad (= APD) \\ L_n \approx L_{n-1} = L \end{cases}$$

- 

$$f_n \approx 1 \quad \Rightarrow \quad g = c_1 - c_0 = \left( A_{Ca} - A_{K1} \right) / A_{Kx} \tag{14}$$

- 

$$x_d \approx 0$$

- 

$$x_n \ll 1 \quad \Rightarrow \quad x_n^2 \approx 0$$

Damit resultiert aus (13 b):

$$\hat{x} = \frac{x_s}{e^{\frac{L}{\tau_x}} - 1} \left( e^{\frac{T}{\tau_x}} - 1 \right) \tag{15}$$

Mit dem Ziel die Abhängigkeit zwischen T und L zu bekommen, also die meßtechnisch bestimmbare *APD*(*HR*)$|_\infty$-Kennlinie formelmäßig zu beschreiben, ist es sinnvoll Beziehung (15) linear zu approximieren. Damit kann T später explizit ausdrücken werden. Linearisiert wird an der Stelle $T=T_1$, wobei hierfür ein physiologisch zu erwartender Wert der Aktionspotentialdauer einzusetzen ist (z.B. $T_1=200$ ms). Es gilt:

$$e^{\frac{T}{\tau_x}} - 1 \approx e^{\theta_1} - 1 + \frac{e^{\theta_1}}{\tau_x} \left( T - T_1 \right) \tag{16 a}$$

und mit den folgenden Substitutionen:

$$\theta_1 = \frac{T_1}{\tau_x}; \qquad \alpha = e^{\theta_1}; \qquad \beta = e^{\theta_1}\left(1 - \theta_1\right) - 1;$$

$$\xi = \frac{x_s}{e^{\frac{L}{\tau_x}} - 1}; \qquad \lambda = \frac{T}{\tau_x}$$

(16 b-f)

resultiert:

$$\hat{x} \approx \xi(\alpha\lambda + \beta) \tag{17}$$

Eingesetzt in (13 a) ergibt:

$$\lambda = \frac{g - b_1\xi(\alpha\lambda + \beta) - b_0}{a_1\xi(\alpha\lambda + \beta) + a_0} \tag{18}$$

oder anders ausgedrückt:

$$\lambda^2 + k_1\lambda + k_0 = 0 \tag{19}$$

mit

$$k_1 = \frac{\beta}{\alpha} + \frac{b_1}{a_1} + \frac{a_0}{a_1\xi\alpha};$$

$$k_0 = \frac{\beta}{\alpha} \cdot \frac{b_1}{a_1} + \frac{b_0 - g}{a_1\xi\alpha}$$

(20 a, b)

Die quadratische Gleichung (19) ist unter Berücksichtigung, daß $\lambda$ reell und $\lambda > 0$ ist, zu lösen. Bei Betrachtung der Koeffizientenwerte und deren Variationsgrenzen unter physiologischen Bedingungen ist die gesuchte Lösung:

$$\lambda = \frac{1}{2}\left(\sqrt{k_1^2 - 4k_0} - k_1\right) \tag{21}$$

Die Gefahr, daß im ungünstigen Fall die Lösung komplexwertig ausfallen kann, bleibt noch bestehen. Diese Frage ist jedoch anhand einer Parameterdiskussion nicht mit vertretbarem Aufwand zu klären. Sie muß daher bei der Entwicklung der Algorithmen berücksichtigt und durch numerische Maßnahmen abgehandelt werden. Durch Rücksubstitution erhält man aus (21):

$$T = \tau_x \left\{ \sqrt{\left[ a\left( e^{\frac{L}{\tau_x}} - 1 \right) + b \right]^2 - \left[ c\left( e^{\frac{L}{\tau_x}} - 1 \right)\left( b_0 - g \right) + d \right]} - \left[ a\left( e^{\frac{L}{\tau_x}} - 1 \right) + b \right] \right\}$$

$$(22)$$

mit

$$a = \frac{1}{2} \cdot \frac{1-\eta}{2\eta-1} e^{-\theta_1} \qquad\qquad b = \frac{1}{2}\left( 1 - \theta_1 - e^{-\theta_1} + \frac{1-\eta}{2\eta-1} - \theta_0 \right)$$

$$c = \frac{1}{2x_s^2} \cdot \frac{e^{-\theta_1}}{\eta(2\eta-1)} \qquad\qquad d = \left( 1 - \theta_1 - e^{-\theta_1} \right) \cdot \left( \frac{1-\eta}{2\eta-1} - \theta_0 \right)$$

woraus schließlich der explizite Zusammenhang zwischen der Aktionspotentialdauer (T) und der Zykluslänge (L) resultiert:

$$T(L, \tau_x, g) = \tau_x \left( \sqrt{ m_1 e^{\frac{2L}{\tau_x}} + m_2 e^{\frac{L}{\tau_x}} + m_3 g e^{\frac{L}{\tau_x}} + m_4 g + m_5 } + m_6 e^{\frac{L}{\tau_x}} + m_7 \right) \qquad (23)$$

mit

$$m_1 = a^2 \qquad\qquad m_5 = (a-b)^2 + cb_0 - d$$
$$m_2 = -2a^2 + 2ab - cb_0 \qquad m_6 = -a$$
$$m_3 = c \qquad\qquad m_7 = a-b$$
$$m_4 = -c$$

Parameter dieser Beziehung sind $\tau_x$ die Zeitkonstante des dynamischen K-Kanals, und $g$, ein Ausdruck der aufgrund von (14) und (10 b, c) die Verhältnisse zwischen den Permeabilitäten des Ca- und der K-Kanäle beschreibt.

Es ist damit gelungen auf Parameter von genau den Ionenkanälen zurück zu rechen, deren Schlüsselrolle im Prozeß der Arrhythmogenese bereits durch experimentelle elektrophysiologische Studien bestätigt wurde [[7]].

Gefragt ist jedoch nicht die Aktionspotenrialdauer selbst als Funktion der zugehörigen Zykluslänge - genau diese Größen sind meßbar und damit bekannt - sonder die physiologischen Parameter $\tau_x$ und $g$ die den Zustand des Substrats charakterisieren.

Die nichtlineare und transzendente Verknüpfung dieser Größen in Beziehung (23) erschwert allerdings das Vorhaben, deren Werte zu bestimmen.

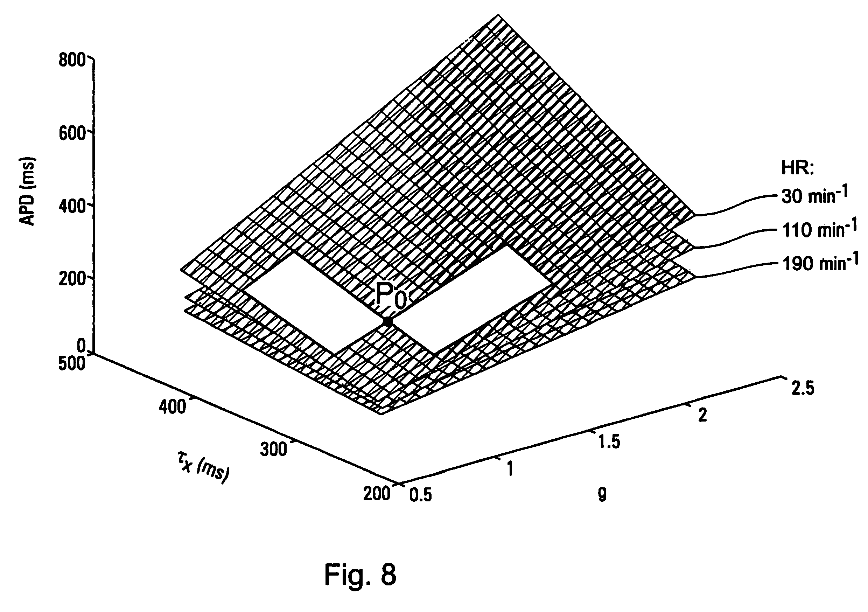

Fig. 8 veranschaulicht die Abhängigkeit der Aktionspotentialdauer T von den physiologischen Parameter $\tau_x$ und $g$ in der Form einer Flächenschaar, deren Schaarparameter die Stimulationsfrequenz ist. An der Tangentialebene im Punkt $P_0 = (\tau_{x0}, g_0)$ ist zu erkennen, daß diese Flächen zwar geringfügig gekrümmt sind, aber auch eine linearisierte Beschreibung diesen Zusammenhang in guter Näherung approximiert. Diese Tatsache rechtfertigt es, im folgenden Beziehung (23) hinsichtlich der Parameter $\tau_x$ und $g$ zu linearisieren. Dann nämlich können deren Werte basierend auf einem linearen Gleichungssystem berechnet werden.

Dazu ist es notwendig, die partiellen Ableitungen der Funktion $T(L, \tau_x, g)$ bezüglich der Parameter $\tau_x$ und $g$ zu ermitteln:

$$\left.\frac{\partial T}{\partial \tau_x}\right|_{\tau_{x0},g_0} = \left(\sqrt{m_1 e^{\frac{2L}{\tau_{x0}}} + m_2 e^{\frac{L}{\tau_{x0}}} + m_3 g_0 e^{\frac{L}{\tau_{x0}}} + m_4 g_0 + m_5} + m_6 e^{\frac{L}{\tau_{x0}}} + m_7\right) +$$

$$- \frac{L}{\tau_{x0}} \left(\frac{2 m_1 e^{\frac{2L}{\tau_{x0}}} + m_2 e^{\frac{L}{\tau_{x0}}} + m_3 g_0 e^{\frac{L}{\tau_{x0}}}}{2\sqrt{m_1 e^{\frac{2L}{\tau_{x0}}} + m_2 e^{\frac{L}{\tau_{x0}}} + m_3 g_0 e^{\frac{L}{\tau_{x0}}} + m_4 g_0 + m_5}} + m_6 e^{\frac{L}{\tau_{x0}}}\right)$$

$$(24\ a,\ b)$$

$$\left.\frac{\partial T}{\partial g}\right|_{\tau_{x0},g_0} = \tau_{x0} \frac{m_3 e^{\frac{L}{\tau_{x0}}} + m_4}{2\sqrt{m_1 e^{\frac{2L}{\tau_{x0}}} + m_2 e^{\frac{L}{\tau_{x0}}} + m_3 g_0 e^{\frac{L}{\tau_{x0}}} + m_4 g_0 + m_5}}$$

sowie den Wert der Funktion im Punkt $P_0 = (\tau_{x0},\ g_0)$:

$$T_0(L) = \tau_{x0} \left(\sqrt{m_1 e^{\frac{2L}{\tau_{x0}}} + m_2 e^{\frac{L}{\tau_{x0}}} + m_3 g_0 e^{\frac{L}{\tau_{x0}}} + m_4 g_0 + m_5} + m_6 e^{\frac{L}{\tau_{x0}}} + m_7\right) (25)$$

In linearisierter Form lautet damit der Zusammenhang zwischen der Aktionspotentialdauer und der Zykluslänge:

$$T(L, \tau_x, g) \approx T_0(L) + \left.\frac{\partial T}{\partial \tau_x}\right|_{\tau_{x0},g_0} \cdot (\tau_x - \tau_{x0}) + \left.\frac{\partial T}{\partial g}\right|_{\tau_{x0},g_0} \cdot (g - g_0) \qquad (26)$$

Diese Beziehung stellt im folgenden die Grundlage für die Entwicklung eines Algorithmus zur Bestimmung der Parameter $\tau_x$ und $g$ dar, also der Größen, die Informationen über den Funktionszustand der Ca- und K-Kanäle hinsichtlich einer drohenden Arrhythmie liefern.

## 5 Meßvorschriften und Entwurf eines Algorithmus zur Parameterextraktion

### 5.1 Definition der Meßgrößen

Wie bereits erwähnt, sind die zu messenden Größen die Zykluslänge L und die Repolarisationszeit T (ganz allgemein gesehen). Diese sind über einen Zeitabschnitt von mehreren Herzschlägen (mindestens 2) aus dem intrakardialen Meßsignal zu extrahieren. Daraus werden M Wertepaare:

$$\left(L_m, T_m\right) \qquad\qquad (27)$$

gebildet. Der Index m bezeichnet die laufende Nummer innerhalb des Wertesets (m= 1..M). Nach dem momentanen Erkenntnisstand sind folgende Anforderungen an das Meßsignal zu stellen:

- **Abtastfrequenz:**
  Eine Abtastfrequenz von 1000 Hz ist insbesondere im Hinblick auf die Algorithmen zur Signalvermessung anzustreben. Geht man aber davon aus, daß der Meßfehler hinsichtlich der Verteilung durch weißes Rauschen genähert werden kann, so ist auch eine Abtastfrequenz von mindestens jedoch 500 Hz noch akzeptabel. Ohnehin wird wie anschließend beschrieben das Gleichungssystem zur Bestimmung der Parameter nach der Methode der kleinsten Fehlerquadrate gelöst. D.h. es werden eine größere Anzahl von Meßwertpaaren verrechnet, wobei sich Fehler (weißes Rauschen) ausmitteln. Strebt man eine Optimierung der Verfahren hinsichtlich der Rechenzeit an, ist eine möglichst genaue zeitliche Auflösung des Signals von Vorteil, da sich damit der Aufwand für die Signalvorverarbeitung verringert.

- *Amplitudenauflösung:*
  An sich ist zwar nur die zeitliche Information von Interesse. Da jedoch die Repolarisationszeit (aus dem noch nicht näher spezifizierten Signal) durch entsprechende Signalverarbeitung ermittelt werden muß, kann diese Frage nur dann genauer beantwortet werden, wenn sowohl das verwendete Meßsignal als auch die Auswertealgorithmen feststehen. Dazu sind jedoch erst eine Reihe klinischer Messungen als Grundlage zur Entwicklung dieser Algorithmen notwendig. Es muß anhand der Signale, die überhaupt durch das Implantat praktisch erfaßt werden können (z.B. IEGM, Coronaris-Sinus-Signal) geklärt werden, welcher Signaltyp sich am besten eignet. Da allerdings auch die Amplitudenauflösung die Genauigkeit bei der Bestimmung des Repolarisationzeitpunktes beeinträchtigt, kann jetzt schon gesagt werden, daß eine Auflösung von unter 8 bit nicht brauchbar ist.

Berücksichtigt man Forderung (14) so sind die Zeitintervalle, während denen die Meßwertpaare ($L_m$, $T_m$) erfaßt werden, so zu wählen, daß so gut es möglich ist, ein eingeschwungener Zustand vorliegt. Dieses ist zwar anhand der Variabilität der Herzrate leicht festzustellen, doch wie die später vorgestellten Simulationsergebnisse zeigen, ist es schon hinreichend, wenn sich die Meßgrößen nicht all zu plötzlich ändern (Änderungsgeschwindigkeit der Herzrate < 10 bpm/ Herzschlag), um dennoch gute Ergebnisse zu erhalten.

### 5.2 Aufbau und Lösung des Gleichungssystems

Ausgehend von den Meßwertpaaren ($L_m$, $T_m$) wird unter Verwendung von (26) das folgende lineare Gleichungssystem aufgebaut:

$$u_{0,m}\tau_x + v_{0,m}g = w_{0,m}: \qquad m = 1\ldots M; \quad M \geq 2 \qquad\qquad (28\text{ a})$$

mit

$$u_{0,m} = \left.\frac{\partial T}{\partial \tau_x}\right|_{\tau_{x0},g_0} = u_0(L_m)$$

$$v_{0,m} = \left.\frac{\partial T}{\partial g}\right|_{\tau_{x0},g_0} = v_0(L_m) \qquad\qquad (28\text{ b, c, d})$$

$$w_{0,m} = w_0(L_m) = T_m - T_0(L_m) + u_0(L_m)\tau_{x0} + v_0(L_m)g_0$$

dessen Lösung den Parametersatz ($\tau_x$, $g$) ergibt. Dafür sind 2 Meßwertpaare ($L_m$, $T_m$) erforderlich, die zu linear unabhängigen Gleichungen führen. Wegen den erwähnten Grenzzyklen der Aktionspotentialdauer und da eine Messung unvermeidlich auch fehlerbehaftet ist, besteht die vorteilhafte Alternative, mehr als nur 2 Meßwertpaare herzunehmen (z.B. M=100). Damit ist (28) ein überbestimmtes Gleichungssystem, dessen Lösung im Sinne der kleinsten Fehlerquadrate erfolgt. Daraus ergibt sich implizit eine Mittelung, so daß Ausreißer die Parameterbestimmung nicht signifikant beeinträchtigen. Hiermit erklärt sich auch, warum sich die im vorigen Punkt gestellte Forderung nach Stationarität der Meßsignale relativiert, nämlich weil Überschwinger infolge eines plötzlichen Frequenzsprunges wie auch Ausreißer ausgemittelt werden.

### 5.3 Iterative Gestaltung des Algorithmus

Eine weitere Verbesserung erzielt man durch eine iterative Gestaltung des Algorithmus zur Parameterextraktion. Für die Aufstellung und Lösung des Gleichungssystems (28) ist es ohnehin erforderlich, eine Schätzlösung ($\tau_{x0}$, $g_0$) für die Parameter, d.h. den Berührungspunkt $P_0$ der Tangentialebene, vorzugeben. Diese Werte können aus physiologischen Überlegungen bzw. aus der Erfahrung hergeleitet werden. Da die Tangentialebene die Fläche in Fig. 8 auch über weitere Bereiche hinweg noch gut nähert, können in nullter Näherung für ($\tau_{x0}$,$g_0$) die dem gesunden Zustand entsprechenden Werte eingesetzt werden, ohne daß die Genauigkeit der Lösung extrem beeinträchtigt wird.

Dennoch ist es vorteilhaft die Genauigkeit der Lösung iterativ zu verbessern. Ausgehend von der Startlösung ($\tau_{x0}$,$g_0$) werden die Koeffizienten des Gleichungssystems (28) bestimmt, und daraus die Lösung ($\tau_x$, $g$) ermittelt. Diese dient nun als Startlösung für die nächste Iteration. Als Abbruchkriterien dienen zwei Fakten: entweder unterscheiden sich die Lösungen (im Sinne der $L_2$-Norm) um weniger als ein geforderter Schwellwert, oder die Anzahl der Iterationen überschreitet einen vorgegebenen akzeptierbaren Wert. Durch das zweite Kriterium ist insbesondere sichergestellt, daß bei divergentem Verhalten, der Algorithmus nicht in eine Endlosschleife führt. Bricht die Iteration aufgrund des zweiten Kriteriums ab, liegt keine Lösung vor. Wie bereits bei der Diskussion von Beziehung (21) erwähnt, die hier in der Form (28 d) in Erscheinung tritt, ist jetzt noch darauf zu achten, daß die Lösung nicht komplexe Werte annimmt. Ist dieses nämlich infolge eines ungünstigen Meßdatensatzes der Fall, wurde keine physiologisch sinnvolle Lösung für $\tau_x$ und $g$ gefunden, so daß ein neuer Meßdatensatz gewählt werden muß.

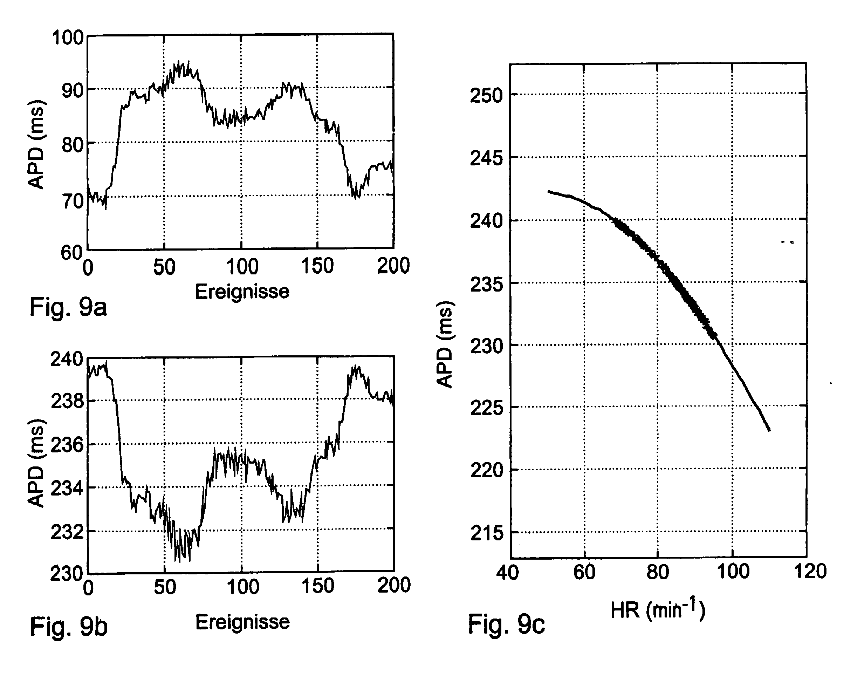

Abschließend visualisieren Fig. 9a, 9b und 9c ein Anwendungsbeispiel dieses Parameter-schätzalgorithmus. Die Fig. 9a und 9b zeigen zum einen die Herzrate (HR) (hier stellvertretend für L), für die in der Simulation ein zufälliger jedoch physiologisch vertretbarer Verlauf angenommen wurde. Zum anderen wurde die Repolarisationszeit T (hier in Form der APD) basierend auf der Modellbeschreibung (13) simuliert. Die Anfangswerte der Zustandsgrößen entsprechen einem eingeschwungenen Zustand bei einer Stimulationsfrequenz von 70 bpm ($x_0$=0.0348, $f_0$=0.9999, $T_0$=239,5 ms).

Diese beiden Trendgrößen (HR und T), die in der klinischen Praxis zunächst aus einem geeigneten intrakardialen Meßsignal als Zahlenfolgen extrahiert werden müssen, stellen die Eingangsgrößen des Schätzalgorithmus dar. In der Fig. 9c ist die $APD(HR)|_\infty$ -Kennlinie aufgetragen, die aus Beziehung (23) resultiert. Parameter dieser Kurve sind die zurückgerechneten Zellparameter ($\tau_x$,$g$). Daher vermittelt die Morphologie dieser Kennlinie Aussagen über den zellulären Funktionszustand.

Um noch einen Einblick quantitativer Natur zu vermitteln, sei das folgende Zahlenbeispiel genannt. Im konkreten Fall (Fig. 9a, 9b und 9c) wurden zur Vorwärtssimulation nach (13) die Parameter $\tau_x$=158 ms und $g$ =0.65 vorgegeben, während als Startwerte für die iterative Rückrechnung $\tau_{x0}$=200 ms und $g_0$=0.5 galten. Als Ergebnis der Parameterschätzung wurden die Werte $\tau_x$ = 160.6788 ms und $g$ =0.6366 innerhalb von 3 Iterationen erhalten.

## 6 Modellrechnungen zum Test des Verfahrens

Um modelltechnisch die Leistungsfähigkeit des beschriebenen Algorithmus nachzuweisen, werden im folgenden als Grundlage einer quantitativen Beurteilung adäquate Testumge-bungen konstruiert. Dabei wird die Parameterrückrechnung sowohl im Bezug auf das Ersatzmodell wie auch auf das physiologisch vollständige Zellmodell untersucht.

### 6.1 Test des Verfahrens am Ersatzmodell

Ausgehend vom Ersatzmodell (13) wurden mehrfach Approximationen vorgenommen, so daß zunächst die Genauigkeit des Verfahrens geprüft werden muß. Vorgenommen wird dieses am Ersatzmodell, da allein daran diese Frage unter definierten Randbedingungen (das Ersatzmodell war Ausgangspunkt für den Entwurf des Parameterschätzverfahrens) beurteilt werden kann.

Für die Tests werden sowohl stochastische als auch deterministische Herzratenverläufe vorgegeben. Die Ermittlung der zugehörigen APD-Werte erfolgt aufgrund der Rekursionsformeln aus (13) und den gleichen Anfangswerten wie im obigen Abschnitt. Die Herzrate startet daher jeweils von 70 bpm, wobei sich deren Variation bei diesen Tests auf $70\pm10$bpm beschränkt. Hintergrund dieser Maßnahme ist zu prüfen, in wie fern das Verfahren auch bei geringer Auslenkung des Systems funktioniert, d.h. also zu klären ob natürliche Variationen der Herzrate (auch ohne explizite Belastung des Patienten) schon ausreichend sind.

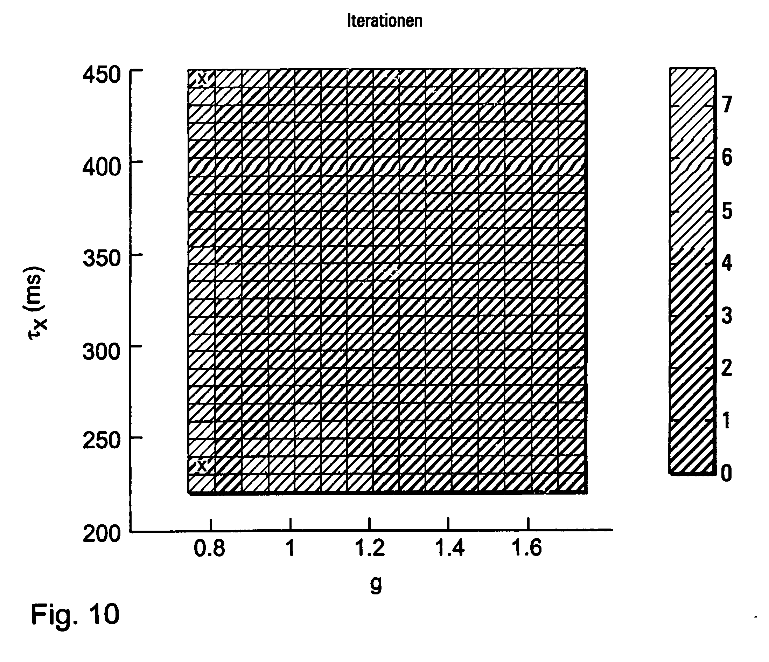

Die Simulationen wurden systematisch für zahlreiche vorgegebene Paramterkombinationen ($\tau_x$, $g$) durchgeführt, deren Variationsbereich (basierend auf Werten der Literatur [[15]]) und Raster aus Fig. 10 zu entnehmen sind.

Zunächst wurde das Verhalten bei einem deterministisch vorgegebenen Herzratenverlauf in Form einer Rampe zwischen 60 bpm und 80 bpm getestet. In der Praxis bedeutet dies, daß der Schrittmacher ein solches Stimulationsprotokoll befolgen muß, um das System auszulenken. Unter diesen Rahmenbedingungen zeichnet sich die Parameterrückrechnung durch relative Fehler unterhalb 2% aus, während überwiegend nur 3 Iterationen benötigt werden.

Im Falle des stochastisch stimulierten Systems muß mit relativen Fehlern von bis zu 5% gerechnet werden, wobei es gelegentlich auch vorkommen kann, daß aufgrund eines gerade ungünstigen Meßwertsatzes, kein physiologisch sinnvolles Ergebnis (z.B. komplexer Wert) erzielt wird. Die Zahl der benötigten Iterationen ist in Abhängigkeit der konkreten Paramterwerte in Fig. 10 dargestellt. Grundsätzlich ist zu bemerken, daß die Bestimmung des Parameters $g$

(auch bei deterministischen Herratenverläufen) weniger fehlerbehaftet ist als die Zeitkonstante $\tau_x$.

Die Simulationsergebnisse zeigten, daß auch bei stochastischen Herzratenverläufen, die den natürlichen nahekommen, noch brauchbare Ergebnisse erzielt werden. Es ist daher nicht unbedingt notwendig, zur Systemidentifikation ein bestimmtes vorgegebenes Stimulationsprotokoll starr zu befolgen, das ggf. sogar den momentanen Bedürfnissen des Organismus nicht entspricht. Das Implantat erfüllt in diesem Sinne überwiegend die Rolle eines diagnostischen Beobachters und veranlaßt nur bei Bedarf eine therapeutische Maßnahme.

### 6.2 Test des Verfahrens am physiologischen Zellmodell

Im nächsten Schritt wird das Verfahren unter realitätsnahen Arbeitsbedingungen untersucht. Als Testobjekt (zu identifizierendes System) dient ein Zellmodell (basierend auf [[6]]), dessen Zustandsgrößen unmittelbar physiologische Größen abbilden. Damit ist es möglich, pathologisches Systemverhalten im direkten Bezug zu klinischen Studien simulationstechnisch nachzubilden.

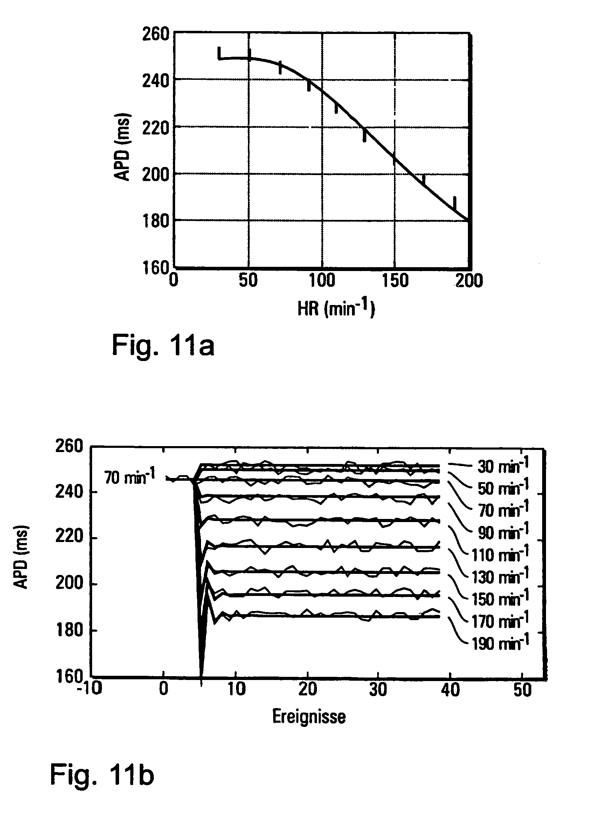

Zunächst wird der grundsätzliche Zusammenhang zwischen den Größen APD und HR untersucht, die Ausgangspunkt der Systemidentifikation sind. Die Figuren 10 und 11 veranschaulichen diesen Zusammenhang für das nun physiologische Zellmodell anhand von zwei Stimulationsprotokollen. In Fig. 11a und 11b wird ähnlich wie in Fig. 3a und 3b die Reaktion des Systems auf eine sprunghafte Veränderung der Stimulationsfrequenz dargestellt. In Fig. 11a wurden die Meßwertpaare (HR, APD) (entsprechend (27)) als Punkte aufgetragen (Gruppen bestehend aus mehreren Meßwertpaaren für jeweils die Stimulationsfrequenz nach dem Sprung). Die durchgezogene Linie stellt hingegen die hieraus (über ($\tau_x$, $g$)) berechnete $APD(HR)|_\infty$ -Kennlinie dar, die das Ergebnis der Systemidentifikation visualisiert. Fig. 11b verbildlicht den Einschwingvorgang selbst: einerseits für das physiologische Zellmodell und andererseite für das Ersatzmodell. Die für das Ersatzmodell notwendigen Parameterwerte ($\tau_x$, $g$) wurden dabei aus den Messungen am physiologischen Modell rückgerechneten. Fig. 11b verdeutlicht insbesondere, wie detailgetreu das Ersatz-modell selbst den Einschwingvorgang nachbildet, also trotz Vereinfachungen auch quantitativ ein physiologisch korrektes Verhalten aufweist.

Als wichtige Aussage von Fig. 11b ist auch festzuhalten, das die Schwingungen der Aktionspotentialdauer kurz nach dem Sprung vorwiegend auf den dynamischen Einschwingvorgang zurückzuführen sind (den selbst das Ersatzmodell wiedergibt), während die Oszillationen im weiteren Verlauf durch den inneren Grenzzyklus verursacht sind. Anders ausgedrückt, klärt damit diese Studie auch, wie ausgeprägt das bereits angesprochene Gedächtnis der Zelle ist, beziehungsweise skizziert sie ein Verfahren, dieses zu messen. Obgleich aus theoretischer Sicht rekursive Systeme ein unendlich langes Gedächtnis haben, ist im konkreten Fall das praktisch meßbare Gedächtnis der Zelle (zumindest im Hinblick auf die hier betrachteten Phänomene) auf etwa 6-7 Zyklen begrenzt. Diese Zahl stellt die Anzahl der signifikanten Überschwinger der APD nach dem Frequenzsprung dar (Fig. 11b).

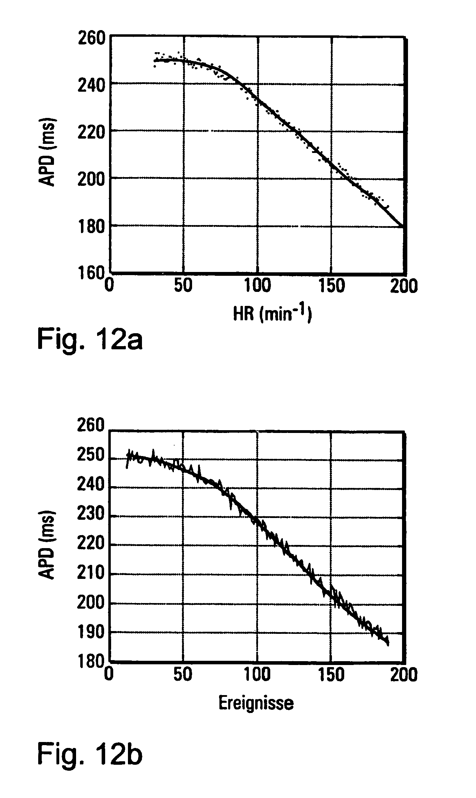

Am Beispiel eines weiteren Tests zeigen Fig. 12a und 12b, daß auch unter anderen Rahmenbedingungen sich das Ersatzmodell physiologisch richtig verhält. Das hier verwendete Protokoll setzt zur Anregung des Systems einen rampenförmig ansteigenden Verlauf der Stimulationsfrequenz ein. Bezüglich der dargestellten Größen sind Fig. 11a, 1 1b und Fig. 12a, 12b analog.

### 6.2.1 Identifikation veränderter Substrateigenschaften unter realitätsnahen Bedingungen

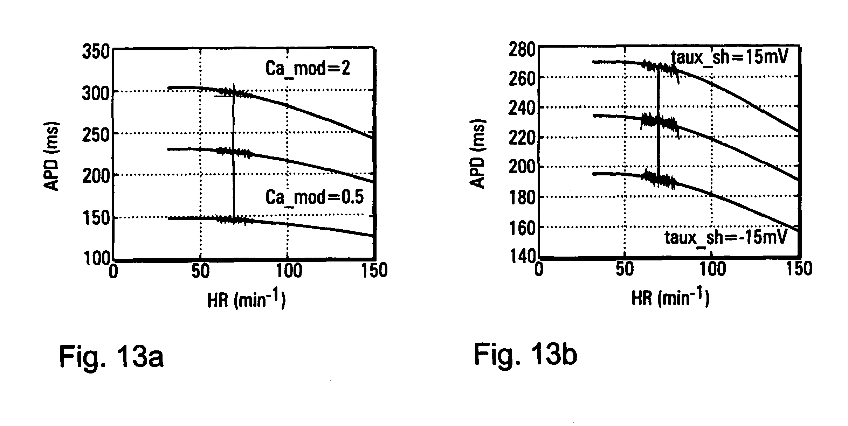

Erwartungsgemäß soll das entwickelte Verfahren auch in der Lage sein, Veränderungen der Substrateigenschaften zu erkennen. Getestet wurde diese Fähigkeit unter Verwendung des physiologischen Zellmodells, wobei konkret die Permeabilität des Ca-Kanals modifiziert und/oder die Relaxationskennlinie $\tau_x(V_m)$ des dynamischen K-Kanals entlang der Abszisse verschoben wurde. Die Tabelle faßt die Größen zusammen, die einerseits die Veränderungen an den Ionenkanälen beschreiben und andererseits die aus den simulierten Messungen bestimmten Systemparameter ($\tau_x$, $g$) wiedergeben. Graphisch sind diese Ergebnisse in Fig. 13a und 13b veranschaulicht.

Die nachfolgende Tabelle zeigt rückgerechnete Parameterwerte für modifiziertes Zellverhalten, wobei Ca_mod die Permeabilität des Ca-Kanals multiplikativ verändert und $\tau_x$ die Verschiebung der Relaxationskennlinie des dynamischen Kanals darstellt.

| Parameter | Bezugswert | Werte für veränderte Zelleigenschaften | | | |
|---|---|---|---|---|---|
| | | Ca_mod=0.5 | Ca_mod=2 | taux_sh=-15mV | taux_sh=15mV |
| $\tau_x$ (mV) | 165.8369 | 173.0361 | 163.9346 | 190.1786 | 158.9006 |
| g | 1.0 | 0.5608 | 1.3717 | 0.6885 | 1.2401 |

Aus der Tabelle ist ersichtlich, daß bei einer Vergrößerung der Permeabilität des Ca-Kanals (Ca_mod>1) aufgrund der Beziehungen (10 b, c) und (14) erwartungsgemäß auch der Wert von $g$ ansteigt. Betrachtet man die Relaxationskennlinie $\tau_x(V_m)$ [[6]], ist ebenfalls festzustellen, daß in Übereinstimmung mit der Tabelle die Werte von $\tau_x$ bei einer Linksverschiebung ansteigen bzw. bei einer Rechtsverschiebung kleiner werden.

Man bemerkt jedoch auch, daß sich die am physiologischen Zellmodell verursachten Modifikationen nicht allein nur auf den einen oder anderen zurückgerechneten Systemparameter auswirken, sondern, daß diese gewisserweise voneinander abhängen. Diese Tatsache ist zwar einerseits den in die Modellbetrachtung eingebrachten Vereinfachungen zuzuschreiben, andererseits aber hängt genau betrachtet $g$ (wegen Beziehung (10 b, c)) von den Verhältnissen der Ca- und K-Stromamplituden ab und nicht von den Leitfähigkeiten selbst. Eine Verschiebung der Relaxationskennline des dynamischen K-Kanals wirkt sich in erster Linie auf den K-Strom selbst aus, aber auch indirekt (über die dadurch veränderte Transmembranspannung) auf den Ca-Strom. Damit wird verständlich, warum sich die Verschiebung der Relaxationskennline auch auf $g$ auswirkt.

Ähnlich läßt sich der andere Zusammenhang begründen. Wird die Permeabilität des Ca-Kanals (über Ca_mod) modifiziert, zieht dieses Veränderungen in der Aktionspotentialform mit sich, die ihrerseits das Relaxationsverhalten des dynamischen K-Kanals beeinflußt. Diese Tatsache führt bei der Parameterrückrechnung zu einem entsprechend anderen Wert von $\tau_x$.

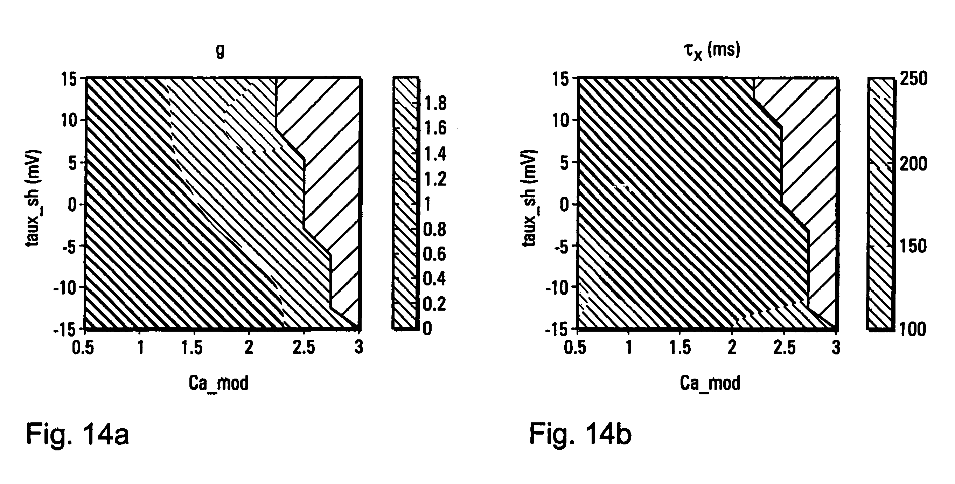

Die Verkopplung der identifizierten Systemparameter ($\tau_x$, $g$) geht aus Fig. 14a und 14b besonders gut hervor, da die Höhenlinien zu keiner der beiden Parameterachsen parallel verlaufen. Diese Graphiken repräsentieren den im Varaitionsbereich der zellulären Modellparameter Ca_mod und taux_sh bestehenden Bezug zu den Systemparametern $\tau_x$ und $g$. Die Intervallgrenzen, innerhalb von denen die Parameter des physiologischen Zellmodells variiert wurden, können aus Fig. 14a und 14b abgelesen werden. Das Raster betrug je 11 Unterteilungen pro Parameterbereich. Zur Paramterschätzung wurden dabei (für jeden Rasterpunkt) je 100 Stimulationszyklen betrachtet.

Für Paramterwerte (Ca_mod, taux_sh) innerhalb der in Fig. 14a und Fig. 14b schraffierten Flächen verhält sich das Zellmodell pathologisch; es treten EADs auf. Da in diesem Extremfall die Modellbetrachtungen zur Herleitung der mathematischen Beziehung zwischen APD und HR nicht mehr gültig sind, ist auch die Anwendung des daraus entwikkelten Verfahrens zur Parameterschätzung in diesem Bereich unzulässig. Daher können im schraffierten Bereich keine Werte für $\tau_x$ und $g$ angegeben werden. In Fig. 14a ist jedoch auffällig, daß der steilste Gradient von $g$ genau in Richtung dieser Gefahrenzone zeigt.

## 7 Anwendung des Verfahrens zur Prädiktion EAD-basierter Arrhythmien

Konsequent umgesetzt führt die aus dem vorigen Abschnitt hervorgehende Schlußfolgerung zur Entwicklung eines Diagnosewerkzeugs, das sowohl für die Früherkennung als auch für die Beobachtung des Fortschreitens zellulärer Erkrankungen bedeutend ist. Von den vielseitig denkbaren Anwendungsgebieten des vorgestellten Identifikationsverfahrens soll hier nur auf die klinische Einsatzmöglkhkeit als EAD-Prädiktor exemplarisch eingegangen werden.

Die Mechanismen und zellulären Ursachen der Arrhythmogenese sind vielschichtig und veränderte Substrateigenschaften können eine Tachykardie auf unterschiedlichste Weise auslösen. Insbesondere wurde klinisch der Zusammenhang zwischen dem Auftreten von EADs und der Reenty-basierten Arrhythmieform 'Torsade de Pointes' nachgewiesen [[10]][[11]]. Andererseits sind diese Mechanismen auch in einer Modellstudie geklärt und nachgebildet worden [[13]]. Damit schließt sich die Argumentationskette, warum dieses Verfahren explizit auch zur Früherkennung von Tachykardien anwendbar ist.

In diesem Sinne kann nun die Frage beantwortet werden, ob das Substrat für eine Arrhythmie prädestiniert ist oder nicht. Streng gesehen ist von Prädiktion nur insofern zu sprechen, daß aus der trendmäßigen Beobachtung der Vergangenheit in die Zukunft extrapoliert werden kann. Das Verfahren selbst liefert jedoch nur Aussagen zum momentanen Ist-Zustand.

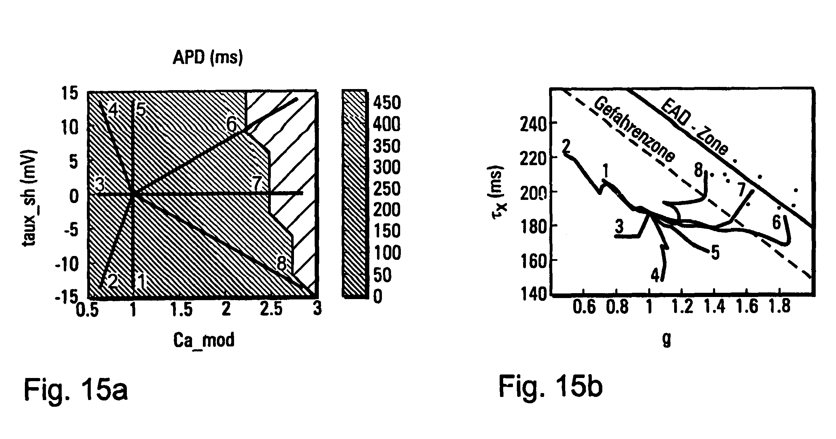

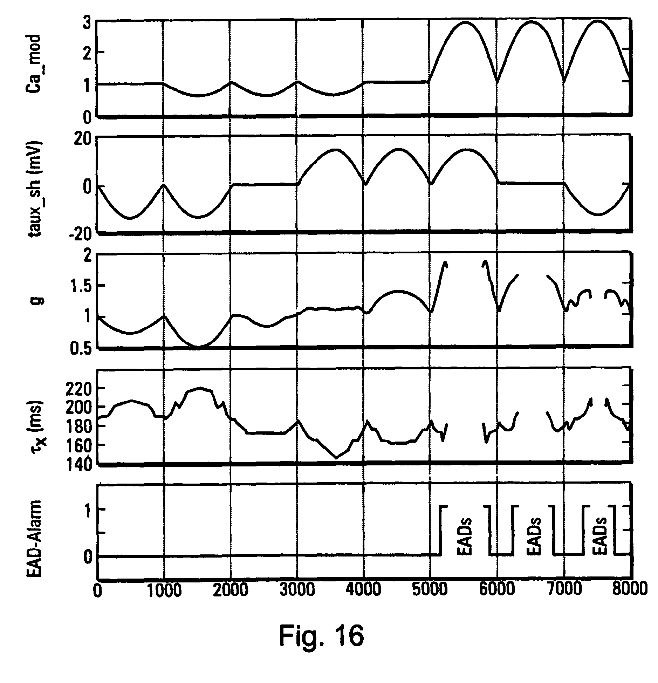

Basierend auf diesen Überlegungen, wird im folgenden das Funktionsprinzip dieses Prädiktors für EAD-bedingte Tachykardien in einer Simulation vorgestellt. Aus Fig. 15a geht der Variationsbereich der zellulären Parameter hervor, die in der Studie die Veränderungen der Substrateigenschaften verursachen. Als Funktionswert ist die entsprechende Aktions-potentialdauer dargestellt. Dem Bild überlagert sind strahlenförmig Trajektorien eingezeichnet (der Ursprung entspricht dem gesunden Normalzustand des Substrats), entlang derer sich in der Modellrechnung die Substrateigenschaften ändern. Wie diese zeitlich durchlaufen werden, zeigt Fig. 16 in den oberen beiden Diagrammen.

Fig. 15b zeigt das Abbild dieser Trajektorien im Parameterraum ($\tau_x$, $g$). Desgleichen wurden die Grenzpunkte des schraffierten EAD-Bereichs aus der linken Abbildung in diesen Paramterraum transformiert und durch diese Punkte eine Regressionsgerade gelegt. Sie stellt im Parameterraum die Grenze zur EAD-Zone dar. In einem (frei definierbaren) Sicherheitsabstand wird parallel dazu eine weitere Gerade gelegt, die die Gefahrenzone markiert. Bewegt sich der Arbeitspunkt ($\tau_x$, $g$) über diese Grenze hinaus, setzt der Algorithmus ein Alarmflag.

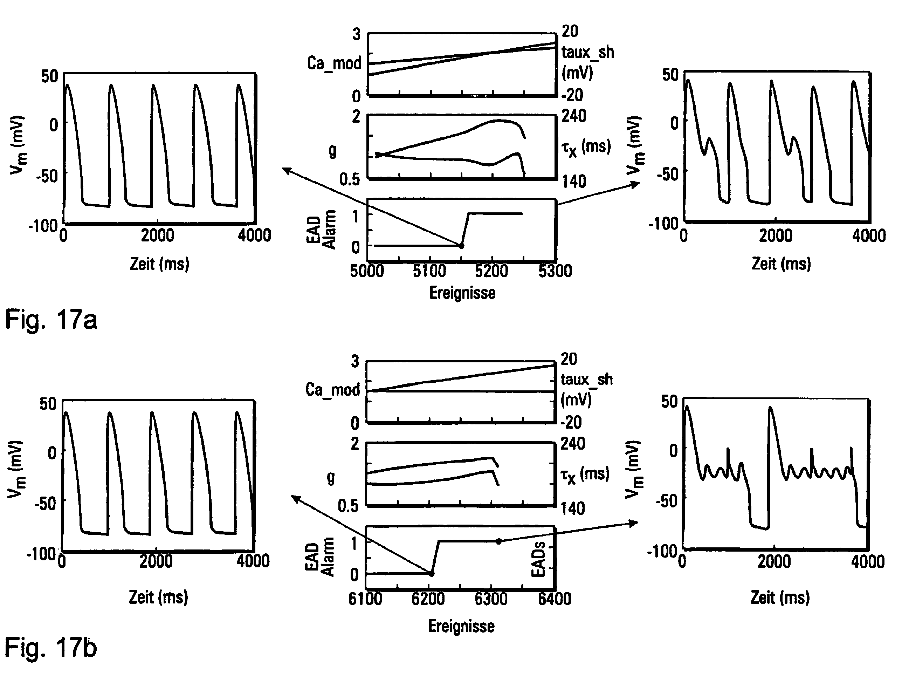

Einen detaillierteren Einblick liefern Fig. 17a und 17b. In zwei Beispielen wird illustriert, wie das Verfahren bereits etwa 100 Herzschläge vor dem Auftreten der EADs diese Gefahr erkennt und meldet. Die genaue Anzahl der Herzschläge hängt im einzelnen allerdings von der Geschwindigkeit ab, mit der sich die Substrateigenschaften ändern. Es ist jedoch mit großer Sicherheit davon auszugehen, daß eine Erkrankung nicht plötzlich von einem Herzschlag zum anderen eintritt, sondern eher allmählich fortschreitet. In der Praxis steht damit erwartungsgemäß ein ausreichender Zeitraum für therapeutische Maßnahmen zur Verfügung.

Wie anhand der beiden Beispiele in Fig. 17a und 17b zu erkennen ist, reagiert das Verfahren nicht nur auf eine EAD-Gefahr, sondern kann bereits im Frühstadium auch das Ausmaß der EADs differenziert beurteilen. Während im oberen Beispiel nur sporadisch EADs auftreten, kommt es im unteren Beispiel aufgrund mehrfacher EADs zu einer schwerwiegenden Verlängerung der Aktionspotentialdauer. An den Spitzen im Verlauf dieser EADs ist zu erkennen, daß eine neue Erregung in die Refraktärzeit trifft, also ein transienter Erregungsblock entsteht. Wie bereits in [[13]] gezeigt, ist in diesem Fall die Entstehung einer kreisenden Erregung viel wahrscheinlicher als im oberen Beispiel.

Obwohl zum Zeitpunkt der Gefahrenmeldung, den Aktionspotentialen rein visuell noch nichts anzumerken ist (Fig. 17a und 17b, linke Diagramme), kann das Verfahren anhand der identifizierten Systemparameter sogar schon feststellen, wie im konkreten Fall die EADs aussehen werden. Hinweise auf EADs liefert zwar auch der Parameter $g$ alleine, dessen Gradient in Richtung der Gefahrenzone am steilsten ist (Fig. 14a und 14b). Die zu erwartenden EAD-Formen können jedoch erst unter Zuhilfenahme des Parameters $\tau_x$ genauer spezifiziert werden.

Wie bereits in [[14]] gezeigt, gilt als notwendiges Kriterium zur Entstehung von EADs, daß die Aktionspotentialdauer drastisch zunimmt. Diese Tatsache wird auch durch Fig. 15a und 15b bestätigt. Als hinreichendes Kriterium kann jedoch nicht einfach ein APD-Schwellwert definiert werden, da dieser entlang der in Fig. 15a eingezeichneten EAD-Grenze um etwa 100 ms variiert. Daher ist die Spezifität des hier entwickelten Verfahrens im Hinblick auf die Arrhythmiegefahr gegenüber derjenigen eines einfachen APD-Kriteriums in jedem Fall überlegen.

## 8 Zusammenfassung

Ausgehend von der mathematischen Beschreibung eines physiologisch begründeten Zellmodells ist es durch vereinfachende Ansätze gelungen, die Grundlage für eine theoretische Systemidentifikation hinsichtlich der Früherkennung von Herzrhythmusstörungen zu schaffen. Im Mittelpunkt der Betrachtungen steht dabei der dynamische Zusammenhang zwischen Herzrate und Repolarisationszeit, dessen Informationsgehalt bezüglich der Arrhythmogenese durch zahlreiche experimentelle Studien der Literatur untersucht und bestätigt wurde.

Als Ergebnis der Systemidentifikation resultierten Rechenvorschriften, die es ermöglichen auf das Amplitudenverhältnis der Ca- und K-Ströme zurückzurechnen sowie die Zeitkonstante des dynamischen K-Kanals zu bestimmen. Es handelt sich dabei um Parameter die einen direkten Bezug zu klinischen Befunden herstellen und sich somit die Arrhythmiegefahr aufgrund von physiologischen Kriterien beurteilen läßt. Die zur Systemanalyse erforderlichen Eingangssignale beschränken sich dabei auf die Herzrate und die Repolarisationszeit, die aus gängigen intrakardialen Meßsignalen (z.B. IEGM) ermittelt werden können.

Zur numerischen Berechnung der definierten Systemparameter wurden im weiteren Implementierungsvorschriften für einen Algorithmus ausgearbeitet, die die Zuverlässigkeit und Genauigkeit bei dennoch vertretbarem Rechenaufwand optimieren.

Als praxisrelevanter Vorteil des entwickelten Verfahrens ist hervorzuheben, daß die zur Systembeobachtung erforderliche Auslenkung des Systems nicht von außen auferlegt werden muß, und damit kein unphysiologischer Eingriff in die Erregungsbildung zu befürchten ist. Die Simulationsergebnisse zeigten, daß sich auch bei natürlichen Herzraten (mit Variationen im Bereich 60-80 bpm) noch aussagekräftige Ergebnisse erzielen lassen. Das Implantat kann damit energiesparend in der Rolle eines diagnostischen Beobachters die Funktionalität des Syncytium überwachen und nur bei Bedarf therapeutische Maßnahmen einleiten. Hinsichtlich der Spezifität kann geschlußfolgert werden, daß dieses Verfahren die Frage der Arrhythmiegefährdung nicht nur binär beantwortet, sondern Aufschlüsse auch darüber liefert, wie schwerwiegend sich die Folgen der veränderten Substrateigenschaften auswirken.

## 9 Literatur

[1] Beeler GW, Reuter H. Reconstruction of the Action Potential of Ventricular Myocardial Fibers. J. Physiol. 1977, 268:177-210

[2] Franz MR, Karasik PL, Li C, Moubarak J, Chavez M. Electrical remodeling of the human atrium: similar effects in patients with chronic atrial fibrillation and atrial flutter. J Am Coll Cardiol, 30(7): 1785-92 1997 Dec

[3] Hnatkova K, Waktare JEP, Murgatroyd FD, Uo X, Baiyan X, Camm AJ, Malik M. Analysis of the Cardiac Rhythm Preceding Episodes of Paroxysmal Atrial Fibrillation. Am Heart J, 1998, 135(6): 1010-1019

[4] Homer SM, Dick DJ, Murphy CF, Lab MJ. Cycle length dependence of the electrophysiological effects of increased load on the myocardium. Circulation, 94(5):1131-6 1996 Sep 1

[5] Homer SM, Murphy CF, Coen B, Dick DJ, Lab MJ. Sympathomimetic modulation of load-dependent changes in the action potential duration in the in situ porcine heart. Cardiovasc Res, 32(1):148-57 1996 Jul

[6] Paule, S. Analyse der Ca-Regulationsmechanismen von Herzmuskelzellen zur Entwicklung antitachykarder Stimulationsmethoden. Diplomarbeit, Friedrich-Alexander-Universität Erlangen-Nürnberg, 1997

[7] Qi A, Tang C, Yeung-Lai-Wah JA, Kerr CR. Characteristics of restitution kinetics in repolarization of rabbit atrium. Can J Physiol Pharmacol, 75(4):255-62 1997 Apr

[8] Taggart P, Sutton PM, Boyett MR, Lab M, Swanton H. Human ventricular action potential duration during short and long cycles. Rapid modulation by ischemia. Circulation, 94(10):2526-34 1996 Nov 15

[9] Unbehauen H. Systemidentifikation mittels Parameterschätzverfahren. In: Unbehauen H. Regelungstechnik III. 5. Auflage, Vieweg Verlag, 1995, 57-132

[10] Verduyn, SC, Vos, MA, Zande, J van der, Kulcsar, A, Wellens, HJJ. Further Observations to Elucidate the Role of Intraventricular Dispersion of Refractoriness and Early Afterdepolarizations in the Genesis of Aquired Torsade de Pointes Arrhythmias. JACC, 1997,30:1575-1584

[11]Volders, PGA, Sipido, KR, Vos, MA, Kulcsar, A, Verduyn, SC, Wellens, HJJ. Cellular Basis of Biventricular Hypotrophy and Arrhythmogenesis in Dogs With Chronic Complete Atrioventricular Block and Acquired Torsade de Pointes. Circulation, 1998, 98:1136-1147

[12] Weiss, I. Meßverfahren und Algorithmen zur Früherkennung von Herzrhythmusstörungen. Interner Bericht, 21.08.98

[13]Weiss I, Urbaszek A, Schaldach M. New Aspects of Arrhythmogenesis Investigated in a Model of EAD-Induced Cardiac Reentry. 19th Annual International Conference of the IEEE EMBS, 1997 (Abstract)

[14] Weiss, I, Urbaszek, A, Schaldach, M. A Model Study of Cardiac Early Afterdepolarizations. Progress in Biomedical Research, December 1996, 84-94

[15]Zeng J, Rudy Y. Early afterdepolarizations in cardiac myocytes: mechanism and rate dependence. Biophysical Journal 1995,68:949-964

**Patentansprüche**

1. Vorrichtung zum vorzeitigen Erkennen einer Tachykardie des Herzens,

   - mit Meßmitteln (12, 16) zum Aufnehmen von Meßwerten für die Herzrate (HR) und die Aktionspotentialdauer (APD), mit mindestens einem Ausgang zum Ausgeben von Meßwertpaaren (HR, APD) einander zugeordneter Meßwerte für die Herzrate (HR) und die Aktionpotentialdauer (APD),
   - mit Meßwertverarbeitungsmitteln (18), die zur Übernahme der Meßwertpaare (HR, APD) mit dem Ausgang der Meßmittel (16) verbunden sind und die zum Ableiten das Herz beschreibender, zeitvarianter Parameter ($\tau_x$, g) aus den Meßwertpaaren (HR, APD) ausgebildet sind,
   - mit einem Speicher (22), in dem eine Tachykardiegefahr kennzeichnende Vergleichswerte speicherbar sind,
   - und mit einer Auswerteeinheit (20), die mit den Meßwertverarbeitungsmitteln (18) zur Übernahme der aus den Meßwertpaaren abgeleiteten Parameter ($\tau_x$, g) und mit dem Speicher (22) verbunden ist und die zum Vergleichen der abgeleiteten Parameter ($\tau_x$, g) mit im Speicher (22) gespeicherten Vergleichswerten und zum Ausgeben eines Tachykardiegefahrensignals ausgebildet ist, falls der Vergleich der abgeleiteten Parameter ($\tau_x$, g) mit den Vergleichswerten ergibt, daß die abgeleiteten Parameter ($\tau_x$, g) in dem Tachykardiegefahrenbereich liegen.

2. Vorrichtung nach Anspruch 1, **dadurch gekennzeichnet, daß** die Vorrichtung zum Aufnehmen mindestens zweier Meßwertpaare (HR, APD) zu verschiedenen Meßzeitpunkten ausgebildet ist und zusätzlich

   - einen zweiten Speicher (24), der mit den Meßwertverarbeitungsmitteln (18) verbunden ist und in dem zumindest die für die zwei jeweils jüngsten Meßzeitpunkte abgeleiteten Parameter ($\tau_x$, g) speicherbar sind sowie
   - Trendbestimmungsmittel, die mit dem zweiten Speicher (24) verbunden sind und die zum Bestimmen eines Trendes für die zukünftige Entwicklung der Parameter ($\tau_x$, g) anhand der gespeicherten Parameter ($\tau_x$, g) ausgebildet sind,

   umfaß und

   daß die Auswerteeinheit (20) mit den Trendbestimmungsmitteln verbunden und so ausgebildet ist, daß sie das Tachykardiegefahrensignal ausgibt, wenn die abgeleiteten Parameter ($\tau_x$, g) in der Nähe des Tachykardiegefahrenbereichs liegen und der Trend für die zukünftige Entwicklung der Parameter in Richtung des Tachykardiegefahrenbereichs weist.

3. Vorrichtung nach Anspruch 1, **dadurch gekennzeichnet, daß** die Vorrichtung zum Aufnehmen mindestens zweier Meßwertpaare (HR, APD) zu verschiedenen Meßzeitpunkten ausgebildet ist und zusätzlich

- einen zweiten Speicher (24), der mit den Meßwertverarbeitungsmitteln (18) verbunden ist und in dem zumindest die für die zwei jeweils jüngsten Meßzeitpunkte abgeleiteten Parameter ($\tau_x$, g) speicherbar sind sowie
- Trendbestimmungsmittel, die mit dem zweiten Speicher (24) verbunden sind und die zum Extrapolieren zukünftiger Parameter aus den gespeicherten Parametern ($\tau_x$, g) ausgebildet sind,

umfaßt und

daß die Auswerteeinheit (20) mit den Trendbestimmungsmitteln verbunden und zum Vergleichen der extrapolierten Parameter mit im ersten Speicher (22) gespeicherten Vergleichswerten und zum Ausgeben eines Tachykardiegefahrensignals ausgebildet ist, falls der Vergleich der extrapolierten Parameter mit den Vergleichswerten ergibt, daß die extrapolierten Parameter in dem Tachykardiegefahrenbereich liegen.

4. Vorrichtung nach einem der Ansprüche 1 bis 3, **dadurch gekennzeichnet, daß** die abgeleiteten Parameter ($\tau_x$, g) zeitvariabele, bidirektionale Ionenströme zwischen dem Zellinneren und dem Zelläußeren von Herzmuskelzellen beschreiben, zu denen ein statischer und eine dynamischer Kalium-Ionenstrom sowie ein Kalzium-Ionenstrom zählen, wobei ein erster der Parameter ($\tau_x$) eine Zeitkonstante des dynamischen Kalium-Ionenstroms ist und ein zweiter der Parameter (g) eine auf die Amplitude des dynamischen Kalium-Ionenstroms ($A_{Kx}$) bezogene Differenz der Amplituden des Kalzium-Ionenstroms ($A_{Ca}$) und des statischen Kalium-Ionenstroms ($A_{Kl}$) ist:

$$g = ((A_{Ca} - A_{Kl}) / A_{Kx})$$

5. Vorrichtung nach einem der Ansprüche 1 bis 4, mit einem Elektrostimulator wie einem Kardioverter oder einem Defibrillator, **dadurch gekennzeichnet, daß** der Elektrostimulator (26) mit der Auswerteeinheit (20) zur Übernahme des Tachykardiegefahrensignals verbunden und durch das Tachykardiegefahrensigrensignal zur Abgabe tachykardierbekämpfender elektrischer Stimulationsimpulse an das Herz auslösbar gestaltet ist.

**Claims**

1. A device for early recognition of a tachycardia of the heart,

- having measurement means (12, 16) for recording measured values for the heart rate (HR) and the action potential duration (APD), having at least one output for outputting measured value pairs (HR, APD) of measured values for the heart rate (HR) and the action potential duration (APD) which are assigned to one another,
- having measured value processing means (18), which are connected to the output of the measurement means (16) for receiving the measured value pairs (HR, APD) and are implemented to derive time-variant parameters ($\tau_x$, g), which describe the heart, from the measured value pairs (HR, APD),
- having a memory (22), in which comparative values characterizing a tachycardia danger are storable,
- and having an analysis unit (20) which is connected to the measured value processing means (18) to receive the parameters ($\tau_x$, g) derived from the measured value pairs and to the memory (22), and which is implemented to compare the derived parameters ($\tau_x$, g) to comparative values stored in the memory (22) and to output a tachycardia danger signal if the comparison of the derived parameters ($\tau_x$, g) to the comparative values shows that the derived parameters ($\tau_x$, g) are in the tachycardia danger range.

2. The device according to Claim 1,
**characterized in that** the device is implemented to record at least two measured value pairs (HR, APD) at different measuring instants and, in addition, it comprises

- a second memory (24), which is connected to the measured value processing means (18), and in which at least the parameters ($\tau_x$, g) derived for the two most recent measuring instants are storable and
- trend determination means, which are connected to the second memory (24) and are implemented to determine a trend for the future development of the parameters ($\tau_x$, g) on the basis of the stored parameters ($\tau_x$, g), and

the analysis unit (20) is connected to the trend determination means and is implemented in such a way that it outputs a tachycardia danger signal when the derived parameters ($\tau_x$, g) are in proximity to the tachycardia danger range and the trend for the future development of the parameters points in the direction of the tachycardia danger range.

3. The device according to Claim 1,
**characterized in that** the device is implemented for recording at least two measured value pairs (HR, APD) at different measuring instants and, in addition, it comprises

- a second memory (24), which is connected to the measured value processing means (18), and in which at least the parameters ($\tau_x$, g) derived for the two most recent measuring instants are storable and
- trend determination means, which are connected to the second memory (24) and are implemented to determine a trend for the future development of the parameters ($\tau_x$, g) on the basis of the stored parameters ($\tau_x$, g), and the analysis unit (20) is connected to the trend determination means and is implemented to compare the extrapolated parameters to comparative values stored in the first memory (22) and to output a tachycardia danger signal if the comparison of the extrapolated parameters to the comparative values shows that the extrapolated parameters lie in the tachycardia danger range.

4. The device according to one of Claims 1 through 3,
**characterized in that** the derived parameters ($\tau_x$, g) describe time-variable, bidirectional ion currents between the cell interior and the cell exterior of cardiac muscle cells, including a static and a dynamic potassium ion current and a calcium ion current, a first of the parameters ($\tau_x$) being a time constant of the dynamic potassium ion current and a second of the parameters (g) being a difference of the amplitudes of the calcium ion current ($A_{Ca}$) and the static potassium ion current ($A_{Kl}$) in relation to the amplitude of the dynamic potassium ion current ($A_{Kx}$):

$$g = ((A_{Ca} - A_{Kl})/A_{Kx}).$$

5. The device according to one of Claims 1 through 4, having an electrostimulator such as a cardioverter or a defibrillator,
**characterized in that** the electrostimulator (26) is connected to the analysis unit (20) for recording the tachycardia danger signal and is designed so it may be triggered by the tachycardia danger signal to output electrical stimulation pulses to the heart which combat tachycardia.

**Revendications**

1. Dispositif pour la détection précoce d'une tachycardie du coeur,

- avec des moyens de mesure (12, 16) pour l'enregistrement de valeurs mesurées pour le rythme cardiaque (HR) et la durée de potentiel d'action (APD), avec au moins une sortie pour la sortie de paires de valeurs mesurées (HR, APD) de valeurs de mesure réciproquement attribuées pour le rythme cardiaque (HR) et la durée de potentiel d'action (APD),
- avec des moyens de traitement de valeurs de mesure (18), qui sont reliés à la sortie des moyens de mesure (16) pour la prise en charge des paires de valeurs mesurées (HR, APD) et qui sont conçus pour la déduction de paramètres ($\tau_x$, g) variant dans le temps et décrivant le coeur à partir des paires de valeurs mesurées,
- avec une mémoire (22), dans laquelle on peut mémoriser des valeurs de comparaison caractérisant un risque de tachycardie,
- et avec une unité d'analyse (20) qui est reliée aux moyens de traitement de valeurs mesurées (18) pour la prise en charge des paramètres ($\tau_x$, g) déduits des paires de valeurs mesurées et à la mémoire (22), et qui est conçue pour la comparaison des paramètres déduits ($\tau_x$, g) avec des valeurs de comparaison mémorisées dans la mémoire (22) et pour la sortie d'un signal de risque de tachycardie, dans le cas où la comparaison des paramètres déduits ($\tau_x$, g) avec les valeurs de comparaison montre que les paramètres ($\tau_x$, g) déduits se situent dans la zone à risque de tachycardie.

2. Dispositif selon la revendication 1, **caractérisé en ce que** le dispositif est conçu pour l'enregistrement d'au moins deux paires de valeurs de mesure (HR, APD) à différents instants de mesure et comprend en supplément

- une seconde mémoire (24), qui est reliée aux moyens de traitements de valeurs mesurées (18) et dans laquelle au moins les paramètres ($\tau_x$, g) déduits pour les deux moments de mesure les plus récents peuvent être mémorisés et
- des moyens de détermination de tendance, qui sont reliés à la seconde mémoire (24) et qui sont conçus pour la détermination d'une tendance pour l'évolution future des paramètres ($\tau_x$, g) à l'aide des paramètres mémorisés ($\tau_x$, g),

et

**en ce que** l'unité d'analyse (20) est reliée aux moyens de détermination de tendance et est conçue de telle sorte qu'elle envoie le signal de risque de tachycardie lorsque les paramètres ($\tau_x$, g) déduits se situent à proximité de la zone à risque de tachycardie et que la tendance pour l'évolution future des paramètres indique la direction de la zone à risque de tachycardie.

3. Dispositif selon la revendication 1, **caractérisé en ce que** le dispositif est conçu pour l'enregistrement d'au moins deux paires de valeurs de mesure (HR, APD) à différents instants de mesure et comprend en supplément

- une seconde mémoire (24), qui est reliée aux moyens de traitement de valeurs mesurées (18) et dans laquelle on peut enregistrer au moins les paramètres ($\tau_x$, g) déduits pour les deux instants de mesure les plus récents et
- des moyens de détermination de tendance qui sont reliés à la seconde mémoire (24) et qui sont conçus pour l'extrapolation de futurs paramètres à partir des paramètres ($\tau_x$, g) mémorisés,

et

**en ce que** l'unité d'analyse (20) est reliée aux moyens de détermination de tendance et est conçue pour la comparaison des paramètres extrapolés avec des valeurs de comparaison mémorisées dans la première mémoire (22) et pour la sortie d'un signal de risque de tachycardie dans le cas où la comparaison des paramètres extrapolés avec les valeurs de comparaison fait apparaître les paramètres extrapolés se situent dans la zone de risque de tachycardie.

4. Dispositif selon l'une quelconque des revendications 1 à 3 , **caractérisé en ce que** les paramètres ($\tau_x$, g) déduits décrivent des courants ioniques variables dans le temps et bidirectionnels entre l'intérieur de la cellule et l'extérieur de la cellule de cellules du muscle cardiaque, au nombre desquels on compte un courant ionique de potassium statique et un courant ionique de potassium dynamique ainsi qu'un courant ionique de calcium, un premier des paramètres ($\tau_x$, g) étant une constante dans le temps du courant ionique de potassium dynamique et un second des paramètres (g) étant une différence, rapportée à l'amplitude du courant ionique de potassium ($A_{Kx}$) dynamique, des amplitudes du courant ionique de calcium ($A_{CA}$) et du courant ionique de potassium ($A_{K1}$) statique :

$$g = ((A_{Ca} - A_{K1}) / A_{Kx})$$

5. Dispositif selon l'une quelconque des revendications 1 à 4, comprenant un électrostimulateur comme un cardioverteur ou un défibrillateur, **caractérisé en ce que** l'électrostimulateur (26) est relié à l'unité d'analyse (20) pour la prise en charge du signal de risque de tachycardie et est conçu de façon à pouvoir être déclenché par le signal de risque de tachycardie pour l'envoi au coeur d'impulsions de stimulation électriques, combattant la tachycardie.

Fig. 1

Fig. 2

Fig. 3b

Fig. 3a

Fig. 4

Fig. 5

Fig. 6

Fig. 7b

Fig. 7a

Fig. 8

Fig. 9c

Fig. 9a

Fig. 9b

EP 1 062 975 B1

Iterationen

Fig. 10

Fig. 11a

Fig. 11b

Fig. 12a

Fig. 12b

Fig. 13b

Fig. 13a

Fig. 14a

Fig. 14b

EP 1 062 975 B1

Fig. 15b

Fig. 15a

Fig. 16

Fig. 17a

Fig. 17b